# EUROPÄISCHE PATENTSCHRIFT

(11) **EP 2 564 196 B1**
(45) Veröffentlichungstag und Bekanntmachung des Hinweises auf die Patenterteilung: **04.11.2015**
(21) Anmeldenummer: 11719501.6
(22) Anmeldetag: 26.04.2011
(51) Int. Cl.: G01N 33/558, G01N 33/94, B01L 3/00

(54) **Mikrofluidiksystem mit Probenvorbehandlung**
Microfluidic system with sample pre-treatment
Système microfluidique doté d'un prétraitement d'échantillons

(30) Priorität: 26.04.2010 EP 10161025
(43) Veröffentlichungstag der Anmeldung: 06.03.2013
(73) Patentinhaber: Securetec Detektions-Systeme AG, 85649 Brunnthal (DE)
(72) Erfinder: ABERL, Franz, 85402 Kranzberg (DE); STADTHAGEN, Torsten, 81379 München (DE); KLAUS, Sebastian, 82061 Neuried (DE)
(74) Vertreter: Weickmann & Weickmann
(86) Internationale Anmeldenummer: PCT/EP2011/056550
(87) Internationale Veröffentlichungsnummer: WO 2011/134946

(56) Entgegenhaltungen:
- EP-A1- 0 634 215
- EP-A2- 0 811 847
- WO-A1-01/35094
- US-B1- 6 203 757

## Beschreibung

Die vorliegende Erfindung betrifft ein Verfahren zur Bestimmung eines Analyten sowie eine hierzu geeignete Vorrichtung.

Immunoassays stellen einen wichtigen Bestandteil klinisch relevanter Analyseverfahren dar, welche auf der Bildung von Immunkomplexen aus Antigenen und Antikörpern basieren und dem Nachweis von Analyten, beispielsweise im Bereich der Medizin-, Umwelt-, Lebensmittel- und Agraranalytik, in flüssigen Proben dienen. Immunoassays bieten aufgrund der biophysikalischen und biochemischen Eigenschaften der Antigen-Antikörper-Bindung im Allgemeinen eine hohe Spezifität und Sensitivität bei vergleichsweise geringem apparativen und wirtschaftlichen Aufwand und weisen insofern signifikante Vorteile gegenüber alternativen Nachweisverfahren auf.

Ein weiteres in der Praxis bedeutendes Anwendungsgebiet von Immunosassays stellt der Nachweis von Drogen in Körperflüssigkeiten oder auf mit Drogen kontaminierten Oberflächen dar. Im Rahmen derartiger Tests wird üblicherweise eine Probe des Analyten mit einem geeigneten Probennahmeelement von einer Oberfläche aufgenommen, das mit dem Analyten benetzte Probennahmeelement mit einem Teststreifen in Kontakt gebracht, und der aus dem Probennahmeelement herausgelöste und auf den Teststreifen transferierte Analyt mittels einer immunologischen Nachweisreaktion detektiert.

Ein Aspekt, welcher speziell beim Nachweis von Drogen (z.B. Amphetamine, Metamphetamine, Cannabis, Kokain, Heroin) eine herausragende Bedeutung besitzt, ist die Spezifität, Sensitivität und Schnelligkeit der verwendeten Tests. Hierbei besteht zum einen das Bedürfnis hinsichtlich hochsensitiver Nachweisverfahren, um selbst im Falle kleiner Probenvolumina bzw. bei Verwendung komplexer Probenmaterialien wie Speichel die Anwesenheit von Drogen zuverlässig und schnell nachweisen zu können. Andererseits sollten die Testformate auch eine hohe Spezifität für die jeweils nachzuweisende Substanz aufweisen, um falsch-positive Messergebnisse auszuschließen und insofern eine verlässliche Aussage darüber zu liefern, um welche konkrete Droge es sich bei der getesten Substanz handelt.

EP 0 699 906 A2 offenbart einen Drogenschnelltest, welcher als Oberflächen-, Schweiß- bzw. Speicheltest unter der Bezeichnung DrugWipe^{®} (Firma Securetec Detektionssysteme AG) kommerziell erhältlich ist. Der Test bedient sich eines im wesentlichen aus Kunststoff bestehenden Wischelements ("Wischer") mit aufgeschweißtem Vlies, mittels welchem eine Probe des Analyten (z.B. von einer Oberfläche oder aus einer Lösung) genommen und anschließend direkt auf einen immunchromatographischen Teststreifen, welcher in einem Einweggehäuse gelagert ist, übertragen wird ("Lateral Flow"-Technologie).

Durch Eintauchen des Teststreifens in eine wässrige Lösung (Wasser oder Puffer mit verschiedenen Reagenzien) wird die Chromatographie gestartet, wobei das Ergebnis der Bestimmung mit dem Auge oder unter Verwendung einer geeigneten Messvorrichtung ausgelesen werden kann. Der Nachweis des Analyten erfolgt dadurch, dass an Drogenmoleküle (Antigene) gebundene Antikörper an einer Testlinie abbinden und aufgrund ihrer Goldmarkierung eine farbige Linie bilden, welche im Auslesefenster optisch detektiert werden kann. Antikörper, welche nicht mit Drogenmolekülen beladen sind, werden vor Erreichen der Testlinie mittels Haptenen, die auf dem Teststreifen in immobilisierter Form vorliegen, abgefangen.

Im Vergleich zu allen anderen auf dem Markt befindlichen Drogenschnelltests sind DrugWipe^{®} und Triage^{®} (Firma Biosite) die beiden einzigen Produkte, welche bei Vorhandensein eines bestimmten Analyten in einer Probe eine farbige Linie ausbilden und insofern eine sogenannte Positivanzeige aufweisen. Alle übrigen kommerziellen Produkte verfügen über eine Negativanzeige, bei der das Nicht-Erscheinen einer Linie als positiver Nachweis für den jeweiligen Analyten gewertet wird. Ein weiterer großer Vorteil des DrugWipe^{®} ist das geringe Probenvolumen, welches zum Nachweis von Drogen und anderen Substanzen benötigt wird. Während im Falle von DrugWipe^{®} ein Probenvolumen von etwa 1-20 µl ausreichend ist, erfordern andere kommerzielle Tests ein Probenvolumen von mindestens 100 µl bis hin zu mehreren Millilitern.

Der von der Firma Varian entwickelte Schnelltest OraLab^{®} 6 dient zum Nachweis von Drogen aus Speichelproben und basiert ebenfalls auf der Lateral-Flow-Technologie. Während die Spezifität des Tests bei etwa 90-100% liegt, beträgt die Sensitivität bei Amphetaminen und Opiaten lediglich zwischen 50 und 90%, bei Δ⁹-Tetrahydrocannabinol und Kokain zum Teil sogar deutlich unter 50% (siehe DRUID-Studie, http://www.druid-project.eu). Ein großer Nachteil dieses Tests liegt weiterhin darin, dass relativ große Probenvolumina benötigt werden, welche bei akuten Drogenkonsumenten oft nicht zur Verfügung stehen.

Der Schnelltest DrugCheck^{®} 5000 der Firma Dräger, welcher ebenfalls auf der Lateral-Flow-Technologie basiert, stellt gemäß den Daten der DRUID-Studie (http://www.druid-project.eu) ein brauchbares Testsystem zum Nachweis von Drogen dar. Allerdings besitzt dieses Testformat den signifikanten Nachteil, dass die Ergebnisse auf dem Teststreifen nur mittels eines Auslesegeräts, welches in der Anschaffung sehr teuer und für den harten Feldeinsatz im Freien nur teilweise geeignet ist, ausgewertet werden können. Insofern ergeben sich für diesen Test erhebliche Probleme in Bezug auf Wirtschaftlichkeit und einfache Handhabbarkeit.

Der von der Firma Mavand angebotene Testkit Rapid STAT^{®} stellt einen weiteren kommerziell erhältlichen Drogenschnelltest dar, bei welchem eine mit Puffer verdünnte Speichelprobe auf einem immunchromatographischen Teststreifen mit markierten Bindungspartnern inkubiert wird und welcher laut Herstellerangaben einen Nachweis bis zu einer unteren Grenze von 15 ng/ml an Δ⁹-Tetrahydrocannabinol ermöglicht.

Ein signifikanter Nachteil dieses Tests ist dessen äußerst komplexe und umständliche Handhabung, welche ihn gerade für eine Anwendung bei der Verkehrspolizei ungeeignet macht, sowie die vergleichsweise geringe Spezifität für Δ⁹-Tetrahydrocannabinol von 80-90% (siehe DRUID-Studie, http://www.druid-project.eu). Laut einer Studie der Universität Mainz liegt die Rate von falsch-positiven Testergebnissen für Δ⁹-Tetrahydrocannabinol bei mehr als 10% und die Gesamtspezifität bei 84% (veröffentlicht auf der GTFCH 2009, Mosbach, http://www.gtfch.org/cms/images/stories/media/tk/ tk76_2/abstractsposter.pdf).

US 7,090,803 B1 und US 7,507,374 B2 (bzw. US 2006/0292035 A1) offenbaren jeweils Vorrichtungen zur Bestimmung eines Analyten in einer Speichelprobe, welche ein Gehäuse zur Aufnahme eines Probennahmeelements, eine Haltevorrichtung zur Aufnahme mindestens eines chromatographischen Teststreifens, eine erste Kammer zur Lagerung eines ersten Reagenzes wie beispielsweise einer Pufferlösung, eine zweite Kammer zur Lagerung eines zweiten, analytspezifischen Reagenzes, Mittel zum Inkontaktbringen der Probe mit dem ersten Reagenz, Mittel zum Inkontaktbringen des Gemisches aus Probe und erstem Reagenz mit dem zweiten Reagenz in der zweiten Kammer, sowie Mittel zum Inkontaktbringen des Gemisches aus Probe, erstem Reagenz und zweitem Reagenz mit dem chromatographischen Teststreifen umfassen.

Im Einzelnen wird hierbei ein Probennahmeelement, welches an einem Ende ein Schwammmaterial mit einer darin aufgenommenen Probe des Analyten umfasst, über eine zylinderförmige Aussparung in das Gehäuse eingebracht und die Probe des Analyten durch Hineindrücken des Probennahmeelements in die Aussparung aus dem Schwammmaterial gepresst. Die Probe gelangt dabei durch eine am Boden der Aussparung befindliche Öffnung in die zweite Kammer, in welcher sie mit dem ersten Reagenz, welches durch Inkontaktbringen mit einem scharfkantigen Gegenstand aus einer deformierbaren Hülle freigesetzt wird und durch eine am Boden der ersten Kammer befindliche Öffnung in die zweite Kammer gelangt, und dem zweiten Reagenz vermischt wird. Das Gemisch wird anschließend für einige Minuten inkubiert und alsdann mit dem chromatographischen Teststreifen in Kontakt gebracht, wobei letzterer eine visuelle Bestimmung des Testergebnisses ermöglicht.

Die in US 7,090,803 B1 und US 7,507,374 B2 offenbarten Vorrichtungen bzw. Verfahren besitzen den Nachteil, dass die Probe des Analyten lediglich mechanisch durch Hineindrücken des Probennahmeelements in die zylinderförmige Aussparung der Testvorrichtung aus dem Schwammmaterial gepresst wird. Auf diese Weise gelangt zum einen nur ein geringer Teil der ursprünglich aufgenommenen Probe in die zweite Kammer, in welcher die Probe mit dem ersten und zweiten Reagenz inkubiert wird und der Analyt an seinen analytspezifischen Bindungspartner bindet, was letztlich die Sensitivität der Analytbestimmung verringert. Zum anderen werden aufgrund der aufsaugenden Wirkung des Schwammmaterials große Probenvolumina benötigt, welche in der Praxis in der Regel nicht zur Verfügung stehen.

US 2008/0166820 A1 beschreibt einen Kit zur Bestimmung eines Analyten in einem Körperfluid, welches ein Probennahmeelement zur Aufnahme einer Probe des Analyten, eine Ampulle mit einer darin enthaltenen Pufferlösung, ein Transferelement zum Entnehmen von Lösung aus der Ampulle und zum Überführen der Lösung in eine Analysevorrichtung, sowie eine Analysevorrichtung umfasst. Die Analysevorrichtung umfasst ihrerseits mindestens einen Reaktionsbereich, welcher zur Aufnahme der Pufferlösung ausgebildet ist und einen für den Analyten spezifischen Bindungspartner enthält, einen chromatographischen Teststreifen, sowie Mittel zum Inkontaktbringen des chromatographischen Teststreifen mit der Pufferlösung.

Zur Bestimmung des Analyten wird das Probennahmeelement in die mit Pufferlösung gefüllte Ampulle eingebracht, wobei sich der mit einer Probe des Analyten getränkte Bereich des Probennahmeelements mit der Pufferlösung vollsaugt und der Analyt durch anschließendes Zusammendrücken des Probennahmeelements in die Pufferlösung eluiert wird. Nach Entfernen des Probennahmeelements wird ein Teil der analythaltigen Pufferlösung mittels des Transferelements aus der Ampulle entnommen und in die Analysevorrichtung überführt, in welcher der Analyt für einige Minuten mit dem analytspezifischen Bindungspartner inkubiert und nachfolgend mit dem chromatographischen Teststreifen in Kontakt gebracht wird.

Der in US 2008/0166820 A1 offenbarte Kit besitzt den Nachteil, dass er aufgrund der Vielzahl an einzelnen Bestandteilen umständlich zu handhaben ist und damit für eine sichere Anwendung beispielsweise als Drogenschnelltest für den Einsatz im Feld nicht in Frage kommt. Ferner besteht durch die Notwendigkeit, die analythaltige Pufferlösung aus der Ampulle zu entnehmen und in eine hiervon separate Analysevorrichtung zu überführen, die Gefahr von Probenverlusten bzw. die Gefahr einer Probenkontamination, was letztlich zu einer Verringerung der Sensitivität bzw. Spezifität des Testergebnisses führen kann und die Gesamtzuverlässigkeit des Tests stark einschränkt.

EP 0 634 215 A1 offenbart ein Verfahren zum Nachweis von Analyten in einer Körperflüssigkeit, welches eine im wesentlichen gleichzeitige, an verschiedenen Entnahmestellen gleichmäßige Bestimmung der Analyten auf mehreren Testelementen ermöglicht. Zur Durchführung des Verfahrens wird hierbei eine Vorrichtung verwendet, welche einen Probenaufgabepunkt zum Aufbringen der Probe, mehrere getrennte Probenentnahmezonen, die durch jeweils eine kapillare Transportstrecke mit dem Probenaufgabepunkt verbunden sind, und mehrere Testelemente zur Einzelbestimmung von Analyten umfasst, wobei auf mindestens einer der Transportstrecken eine Verzögerungszone zur Verlangsamung des Transports von Flüssigkeit vom

Probenaufgabepunkt zur Probenentnahmezone vorgesehen ist.

Ein Nachteil des in EP 0 634 215 A1 dargestellten Testformates ist, dass der in der Probe befindliche Analyt nicht in einem vom Testelement separaten Bereich mit einem analytspezifischen Bindungspartner inkubiert wird, nachdem die obligatorisch vorhandene Verzögerungszone keinen analytspezifischen Bindungspartner enthält und lediglich eine Überschwemmung des Tests durch die Probe vermeiden bzw. eine gleichzeitige Benetzung der einzelnen Testelemente mit der Probe sicherstellen soll. Auf diese Weise ist die Basis für eine optimale Kinetik zwischen Analyt und analytspezifischem Bindungspartner nicht gegeben, wodurch Sensitivität und Spezifität, insbesondere beim Nachweis von schlecht wasserlöslichen (hydrophoben) Analyten, deutlich limitiert sind.

EP 0 811 847 A2 offenbart ein Verfahren zum Nachweis eines Analyten in einer Körperflüssigkeit bzw. auf einer kontaminierten Oberfläche. Zur Durchführung des Verfahrens wird hierbei jeweils ein Testkit verwendet, welcher einen Teststreifen aus einem oder mehreren kapillaraktiven chromatografiefähigen Materialien, ein Probennahmeelement separat zur Teststreifenoberfläche, sowie eine Andrückvorrichtung zum Inkontaktbringen von Teststreifenoberfläche und Probennahmeelement umfasst, wobei das Probennahmeelement zwecks besserer Aufnahme des Analyten mit einem wässrigen Puffer, einem Detergenz oder/und einem organischen Lösungsmittel angefeuchtet sein kann.

Der in EP 0 811 847 A2 beschriebene Test hat den Nachteil, dass die den Analyten enthaltende Probe vor Inkontaktbringen mit dem Testelement in keinster Weise aufbereitet wird, was insbesondere bei der Bestimmung von biologischen Proben mit komplexer chemischer Zusammensetzung (z.B. Blut, Speichel oder Schweiß) von Bedeutung ist. Ungeachtet der Tatsache, dass biologische Proben per se einer personenabhängigen Schwankungsbreite unterliegen, hat insbesondere die Beschaffenheit der Probenmatrix (z.B. Viskosität, Proteingehalt, etc.) einen großen Einfluss auf den Analytnachweis, da die Sensitivität des Nachweisverfahrens in hohem Maße von der Zugänglichkeit des Analyten für den analytspezifischen Bindungspartner abhängig ist.

Ein weiterer Nachteil des in EP 0 811 847 A2 beschriebenen Testformates ist, dass der in der Probe befindliche Analyt wiederum nicht in einem vom Testelement separaten Bereich mit einem analytspezifischen Bindungspartner inkubiert wird. Auf diese Weise ist keine optimale Kinetik zwischen Analyt und analytspezifischem Bindungspartner gegeben, wodurch Sensitivität und Spezifität, insbesondere beim Nachweis von schlecht wasserlöslichen Analyten, stark limitiert sind.

US 6,203,757 B1 offenbart ein Verfahren zum Nachweis von Analyten in einer Körperflüssigkeit, welches eine simultane Bestimmung mehrerer Analyten mittels einer einzigen Probe vorsieht. Zur Durchführung des Verfahrens wird eine Testvorrichtung verwendet, welche mindestens zwei, im wesentlichen parallel zueinander angeordnete Teststreifen sowie ein V-förmig oder W-förmig ausgebildetes Probenverteilerelement, das eine fluide Kommunikation zwischen dem Probenaufgabepunkt und den einzelnen Teststreifen ermöglicht, umfasst. In einer Ausführungsform kann es sich bei dem Analyten um ein Drogenmolekül handeln, welches beispielsweise mittels eines enzymatischen Immunoassays detektiert wird.

Ein Nachteil des in US 6,203,757 B1 beschriebenen Nachweisverfahrens ist, dass die auf die Testvorrichtung aufgetropfte Probe vor Inkontakttreten von Analyt und analytspezifischem Bindungspartner entlang eines gewebeartiges Materials transportiert wird. Aufgrund der Tatsache, dass gewebeartige Materialien üblicherweise sehr große Oberflächen aufweisen, an die der Analyt unspezifisch binden kann, steht in derartigen Tests häufig nicht mehr die gesamte in der Probe vorhandene Menge an Analyt für eine Reaktion mit dem analytspezifischen Bindungspartner zur Verfügung. Dies ist beim Nachweis vor allem von schlecht wasserlöslichen Analyten sehr nachteilig und limitiert in manchen Fällen die Sensitivität dramatisch.

Ein weiterer Nachteil des in US 6,203,757 B1 beschriebenen Testformates ist, dass der in der Probe befindliche Analyt ebenfalls nicht in einem vom Testelement separaten Bereich mit einem analytspezifischen Bindungspartner inkubiert wird. Auf diese Weise sind die Sensitivität und Spezifität des Tests, insbesondere beim Nachweis von schlecht wasserlöslichen (hydrophoben) Analyten, stark limitiert, weshalb speziell der Nachweis von Analyten, welche lediglich in geringer Konzentration in der zu untersuchenden Probe vorhanden sind, mit Problemen behaftet ist.

WO 01/35094 A1 offenbart einen Testkit zum Nachweis von Analyten in einer Körperflüssigkeit, welcher aus einem verschließbaren Gehäuse, einem darin gelagerten Sammelbehälter zur Aufnahme von Körperflüssigkeiten und mindestens einem Teststreifen besteht.

Ausgehend von oben beschriebenen Testsystemen bestand die der vorliegenden Erfindung zugrunde liegende Aufgabe somit darin, ein Verfahren zur Bestimmung eines Analyten, insbesondere zur Bestimmung von Drogen, bereitzustellen, bei welchem die Nachteile des Standes der Technik zumindest teilweise beseitigt sind. Insbesondere sollte das Verfahren für alle zu testenden Analyten eine hohe Sensitivität und Spezifität aufweisen, leicht handhabbar sein und eine schnelle Bestimmung ohne den Einsatz zusätzlicher technischer Hilfsmittel zum Auslesen der Testergebnisse ermöglichen.

Diese Aufgabe wird erfindungsgemäß gelöst durch ein Verfahren zur Bestimmung eines Analyten, umfassend die Schritte:
(a) Bereitstellen einer mikrofluidischen Analysevorrichtung, umfassend zumindest einen ersten, einen zweiten und einen dritten Bereich, wobei der erste Bereich mit dem zweiten Bereich und der zweite Bereich mit dem dritten Bereich durch Mikrokanäle, Stufen, Verzweigungen oder/und Kammern verbunden ist,
(b) Aufnehmen einer den Analyten enthaltenden Probe von einer Probenoberfläche mit einem Probennahmeelement,
(c) Einbringen des Probennahmeelements in den ersten Bereich der mikrofluidischen Analysevorrichtung und Eluieren des Analyten in dem ersten Bereich mit einem Elutionsmittel,
(d) Überführen des in Schritt (c) erhaltenen Eluats in den zweiten Bereich der mikrofluidischen Analysevorrichtung und Inkontaktbringen des Eluats mit einem in dem zweiten Bereich gelagerten analytspezifischen Bindungspartner für einen Zeitraum von mindestens 3 Sekunden in Abwesenheit eines zum Nachweis des Analyten dienenden Testelements, wobei eine Bindung des Analyten an den analytspezifischen Bindungspartner erfolgt,
(e) Überführen des in Schritt (d) erhaltenen Gemisches in den dritten Bereich der mikrofluidischen Analysevorrichtung und Inkontaktbringen des Gemisches mit mindestens einem in dem dritten Berich gelagterten, zu Bestimmung des Analyten dienenden Testelement, und
(f) Bestimmen des Vorhandenseins oder/und der Menge des Analyten auf dem Testelement.

Überraschenderweise wurde im Rahmen der vorliegenden Erfindung festgestellt, dass mittels des erfindungsgemäßen Verfahrens Analyten einfach und reproduzierbar mit hoher Sensitivität und Spezifität nachgewiesen werden können, ohne dass hierzu große Probenmengen des Analyten oder/und komplexe technische Hilfsmittel zum Auslesen der Testergebnisse erforderlich sind.

Der erste Schritt des erfindungsgemäßen Verfahrens erfordert die Bereitstellung einer für die Bestimmung des Analyten geeigneten mikrofluidischen Analysevorrichtung, welche zumindest einen ersten, einen zweiten und einen dritten Bereich umfasst. Der erste Bereich der mikrofluidischen Analysevorrichtung ist zum Einbringen eines Probennahmeelements, mittels welchem vorab eine Probe des Analyten aufgenommen wurde, sowie zum anschließenden Eluieren des Analyten von dem Probennahmeelement mit einem Elutionsmittel ausgebildet, während der zweite Bereich einen für den Analyten spezifischen Bindungspartner enthält und mit dem ersten Bereich in fluider Kommunikation steht. Der dritte Bereich umfasst schließlich mindestens ein Testelement zur Bestimmung des Vorhandenseins oder/und der Menge des Analyten und steht mit dem zweiten Bereich in fluider Kommunikation.

Die jeweiligen Bereiche der mikrofluidischen Analysevorrichtung sind durch mikrofluidische Strukturen, wie beispielsweise durch Mikrokanäle, Stufen, Verzweigungen oder/und Kammern, miteinander verbunden und ermöglichen auf diese Weise einen Transfer bzw. eine Prozessierung von Fluiden innerhalb der Analysevorrichtung ermöglichen. Mikrofluidische Strukturen, wie sie vorstehend genannt sind, können durch dem Fachmann bekannte Verfahren, wie beispielsweise durch zerspanendes Fräsen oder Spritzguss, entsprechend den jeweiligen Anforderungen an die Analysevorrichtung aus geeigneten Materialien, insbesondere aus Kunststoff, hergestellt werden.

Zur Bestimmung des Analyten wird eine den Analyten enthaltende Probe im nächsten Schritt des erfindungsgemäßen Verfahrens mittels eines geeigneten Probennahmeelements von einer zu untersuchenden Probenoberfläche (z.B. Zunge, Haut, sonstige Oberfläche) aufgenommen. Als Probennahmeelement kann dabei grundsätzlich jedes beliebige Element verwendet werden, welches eine Probe des Analyten aufzunehmen vermag und diese bei nachfolgendem Kontakt mit einem Elutionsmittel nahezu quantitativ, d.h. in einer Menge von ≥ 95 Gew.% bezogen auf das Gesamtgewicht der aufgenommenen Probe, wieder abgeben kann. Probennahmeelemente, welche für die Zwecke der vorliegenden Erfindung besonders geeignet sind, sind beispielsweise in EP 1 608 268 A1 und WO 2004/086979 A1 beschrieben, auf deren Offenbarung hiermit Bezug genommen wird.

Das Probennahmeelement bzw. ein zur Aufnahme der Probe ausgebildeter Bereich des Probennahmeelements kann grundsätzlich aus einem beliebigen Material bestehen, das dem Fachmann für die Zwecke der vorliegenden Erfindung nützlich erscheint und sowohl eine Anreicherung des Analyten auf dem Probennahmeelement als auch dessen spätere Abgabe bei Inkontaktbringen mit einem Elutionsmittel ermöglicht. Neben den in EP 1 608 268 A1 und WO 2004/086979 A1 beschriebenen Probennahmeelementen kommen insofern Probennahmeelemente in Betracht, welche saugfähige Materialien, insbesondere Gewebe, Vliese oder/und poröse Matrices (z.B. Membranen und Schwämme), umfassen. Geeignete Vliese sind beispielsweise in DE 38 02 366 A1 und EP 0 699 906 A2 beschrieben, auf deren Offenbarung ausdrücklich Bezug genommen wird.

Um eine hohe Sensitivität und Spezifität bei der Bestimmung des Analyten zu gewährleisten, kann die Oberfläche des Probennahmeelements vor erstmaligem Gebrauch chemisch vorbehandelt werden; auf diese Weise ist es möglich, die Aufnahme des Analyten bei der Probennahme zu verbessern oder/und ein Anhaften des Analyten am Probennahmeelement zu minimieren. Insofern sieht das erfindungsgemäße Verfahren in einer bevorzugten Variante vor, dass das Probennahmeelement ein Übertragungsreagenz, enthaltend mindestens ein Protein, mindestens ein Kohlenhydrat, mindestens einen Zuckeralkohol oder/und mindestens ein Salz, insbesondere ein anorganisches Salz, umfasst.

Das Übertragungsreagenz, welches den Transfer des Analyten von der Probenoberfläche auf das Probennahmeelement oder/und die nachfolgende Abgabe des Analyten an das Elutionsmittel insbesondere durch Blockierung freier Bindungsstellen auf dem Probennahmeelement oder/und Beeinflussung der Analyteigenschaften begünstigt, kann zu diesem Zweck beispielsweise auf dem Probennahmeelement imprägniert sein. Techniken, welche zur Aufbringung des Übertragungsreagenzes auf das Probennahmeelement verwendet werden können, sind dem Fachmann allgemein bekannt.

Der Begriff "Kohlenhydrat", wie er in der vorliegenden Anmeldung verwendet wird, bezeichnet Monosaccharide, Oligosaccharide und Polysaccharide der allgemeinen Summenformel CₙH₂ₙOₙ, welche jeweils natürlichen oder synthetischen Ursprungs sein können. Im Rahmen der Erfindung gelangen bevorzugt Monosaccharide oder Oligosaccharide zum Einsatz, wobei als Monosaccharide insbesondere natürlich vorkommende Tetrosen, Pentosen und Hexosen, wie beispielsweise Erythrose, Threose, Ribose, Arabinose, Lyxose, Xylose, Allose, Altrose, Galactose, Glucose, Gulose, Idose, Mannose, Talose und Fructose, die jeweils in der D-Form oder in der L-Form vorliegen können, Anwendung finden. Als Oligosaccharide können insbesondere natürlich vorkommende Disaccharide und Trisaccharide verwendet werden, wie beispielsweise Lactose, Maltose, Saccharose, Trehalose, Gentianose, Kestose und Raffinose. In einer besonders bevorzugten Ausführungsform der Erfindung enthält das Übertragungsreagenz ein Kohlenhydrat ausgewählt aus der Gruppe bestehend aus Glucose, Lactose, Maltose und Saccharose.

Der Begriff "Zuckeralkohol", wie er in der vorliegenden Anmeldung verwendet wird, bezeichnet Monosaccharid-Zuckeralkohole der allgemeinen Summenformel CₙH₂ₙ₊₂Oₙ und Disaccharid-Alkohole der allgemeinen Summenformel CₙH₂ₙOₙ₋₁, welche jeweils natürlichen oder synthetischen Ursprungs sein können. Bevorzugte Monosaccharid-Zuckeralkohole umfassen Glycerol, Erythritol, Threitol, Ribitol, Arabinitol, Xylitol, Allitol, Altritol, Galactitol, Glucitol, Iditol und Mannitol, die jeweils in der D-Form oder in der L-Form vorliegen können. Als Disaccharid-Zuckeralkohole können insbesondere Isomalt, Lactitol und Maltitol verwendet werden. In einer besonders bevorzugten Ausführungsform der Erfindung enthält das Übertragungsreagenz einen Zuckeralkohol ausgewählt aus der Gruppe bestehend aus Glucitol, Glycerol, Mannitol und Xylitol.

Bevorzugt gelangt im Rahmen des erfindungsgemäßen Verfahrens ein Übertragungsreagenz zum Einsatz, welches (a) mindestens ein Protein ausgewählt aus der Gruppe bestehend aus Gelatine, Ovalbumin und Rinderserumalbumin, (b) Magermilchpulver, (c) mindestens ein Kohlenhydrat ausgewählt aus der Gruppe bestehend aus Glucose, Lactose, Maltose und Saccharose, (d) mindestens einen Zuckeralkohol ausgewählt aus der Gruppe bestehend aus Glucitol, Glycerol, Mannitol und Xylitol, oder/und (e) mindestens ein Salz ausgewählt aus der Gruppe bestehend aus Calciumchlorid, Kaliumchlorid, Magnesiumchlorid, Natriumchlorid und einem Borat, umfasst. Besonders bevorzugt umfasst das erfindungsgemäß verwendete Übertragungsreagenz mindestens ein Protein ausgewählt aus der Gruppe bestehend aus Gelatine, Ovalbumin und Rinderserumalbumin, oder/und Magermilchpulver.

Die Konzentration an dem mindestens einen Protein, mindestens einen Kohlenhydrat, mindestens einen Zuckeralkohol oder/und mindestens einen Salz in oben beschriebenem Übertragungsreagenz kann vom Fachmann entsprechend den jeweiligen Anforderungen an den Analyten angepasst werden, beträgt jedoch üblicherweise etwa 0.01 bis etwa 15 Gew.-%, bezogen auf das Gesamtgewicht des Übertragungsreagenzes. Sofern das Übertragungsreagenz Salze enthält, werden diese üblicherweise in Konzentrationen von etwa 1 µM bis etwa 1 M zugesetzt.

Neben dem mindestens einen Protein, mindestens einen Kohlenhydrat, mindestens einen Zuckeralkohol oder/und mindestens einen Salz kann das Übertragungsreagenz gegebenenfalls weitere Reagenzien enthalten, welche einen Transfer des Analyten von der zu untersuchenden Oberfläche auf das Probennahmeelement oder/und die spätere Abgabe des Analyten an das Elutionsmittel begünstigen, wie beispielsweise ein Detergenz oder/und ein organisches Lösungsmittel. Beispiele für Detergenzien umfassen u.a. Cholamidopropansulfonat, Octylglucosid, Polidocanol, Polyalkylenglykolether (z.B. Brij^{®}, Synperonic^{®}) und Polysorbate (z.B. Tween^{®} 20, Tween^{®} 80), welche üblicherweise in einer Konzentration von etwa 0.01 bis etwa 5 Gew.-%, bezogen auf das Gesamtgewicht des Übertragungsreagenzes, zum Einsatz gelangen. Beispielhafte organische Lösungsmittel umfassen insbesondere Dimethylsulfoxid, Ethanol, Methanol, Glycerin, sowie Gemische hiervon, welche dem Übertragungsreagenz in einer Konzentration von üblicherweise < 30 Gew.-% zugesetzt werden.

In einer bevorzugten Variante sieht das erfindungsgemäße Verfahren die Verwendung eines Probennahmeelements vor, welches einen Volumenindikator umfasst. Der Volumenindikator zeigt dem Anwender bei der Probennahme an, ob ein für die Bestimmung des Analyten ausreichendes, definiertes Probenvolumen genommen wurde. Dies ist insbesondere bei der Probennahme von Flüssigkeiten wie z.B. Speichel von entscheidender Bedeutung, da im Allgemeinen nur bei Bereitstellung eines definierten Probenvolumens eine optimale Performance des jeweiligen Testsystems gewährleistet werden kann. Eine negative Beeinflussung des Testsystems durch den Probanden, beispielsweise durch Abgabe eines zu geringen Probenvolumens, kann durch den Volumenindikator verhindert werden, wodurch letztlich die Sensitivität, Spezifität und Gesamtzuverlässigkeit des Testsystems optimiert werden kann.

Besonders bevorzugt handelt es sich bei dem Volumenindikator um einen Farbindikator, welcher bei Kontakt mit einem ausreichenden Probenvolumen, beispielsweise bei Kontakt mit einem ausreichenden Volumen an Körperfluid, seine Farbe ändert und insofern mit dem zur Bestimmung des Analyten erforderlichen Probenvolumen korreliert. Als Farbindikator kann jeder dem Fachmann bekannte und für die Zwecke der vorliegenden Erfindung geeignet erscheinende Farbindikator verwendet werden, solange er die oben genannten Kriterien erfüllt und darüber hinaus nicht toxisch ist. Beispiele für derartige Farbindikatoren umfassen insbesondere gängige pH-Farbindikatoren oder Pflanzenfarbstoffe, welche beispielsweise durch Bedampfen, Bedrucken, Besprühen oder/und Tränken des Probennahmeelements auf letzteres aufgebracht werden können.

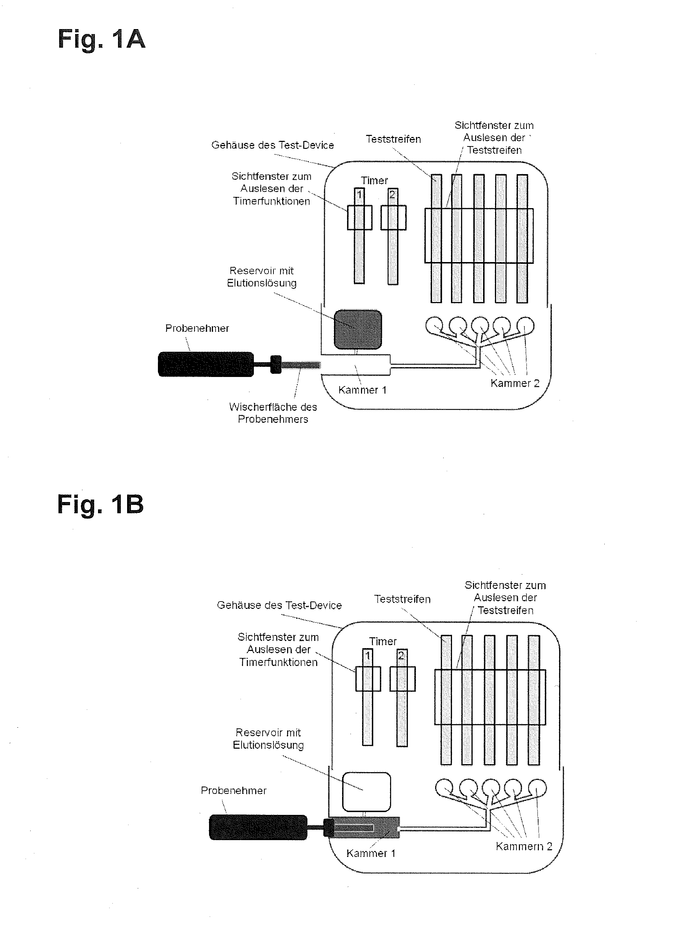

Im nächsten Schritt des erfindungsgemäßen Verfahrens wird das mit der Probe des Analyten benetzte Probennahmeelement in den ersten Bereich der mikrofluidischen Analysevorrichtung, welcher vorzugsweise als Kammer ausgebildet ist, eingebracht (Fig. 1A). Das Probennahmeelement sowie der zur Aufnahme bzw. Integrierung des Probennahmeelements ausgebildete erste Bereich der mikrofluidischen Analysevorrichtung sind hierbei derart ausgestaltet, dass der erste Bereich nach Einbringen des Probennahmeelements dicht verschlossen wird und keinerlei fluide Kommunikation zwischen dem Inneren des ersten Bereichs und der äußeren Umgebung stattfinden kann.

Nachdem das Probennahmeelement in den ersten Bereich der mikrofluidischen Analysevorrichtung aufgenommen worden und die Dichtheit des ersten Bereichs gegenüber der äußeren Umgebung sichergestellt ist, wird Elutionsmittel in den ersten Bereich der mikrofluidischen Analysevorrichtung eingebracht (Fig. 1B), wobei der Analyt aus dem Probennahmeelement eluiert und vorzugsweise homogen im Elutionsmittel verteilt wird. Insofern sieht die Erfindung in einer bevorzugten Variante vor, dass nach Einbringen von Elutionsmittel in den ersten Bereich ein homogenes Gemisch aus Probe bzw. Analyt und Elutionsmittel bereitgestellt wird, was beispielsweise durch eine mikrofluidische Mischerstrecke oder eine andere dem Fachmann bekannte mikrofluidische Mischerstruktur sichergestellt werden kann. Dies hat den Vorteil, dass die Probe vor Inkontaktbringen mit einem geeigneten Testelement zusätzlich aufbereitet wird, wodurch der Analyt für einen analytspezifischen Bindungspartner leichter und effizienter zugänglich ist und Schwankungen insbesondere bei der Bestimmung von biologischen Proben minimiert werden. Demzufolge kann hierdurch die Sensitivität des Verfahrens merklich erhöht werden.

Bei Verwendung einer geeigneten Kombination aus Probennahmeelement und Elutionsmittel kann mittels obiger Verfahrensführung sichergestellt werden, dass die Probe und der darin enthaltene Analyt im wesentlichen quantitativ, d.h. in einer Menge von ≥ 95 Gew.% bezogen auf das Gesamtgewicht der aufgenommenen Probe, aus dem Probennahmeelement eluiert wird, der Analyt im wesentlichen quantitativ aus der Probenmatrix herausgelöst wird und eine vollständige Vermischung von Analyt und Elutionsmittel erfolgt. Ein quantitatives Herauslösen der Probe aus dem Probennahmeelement sowie ein quantitatives Herauslösen des Analyten aus der Probenmatrix ist für eine maximale Sensitivität diagnostischer Testsysteme von essentieller Bedeutung, da als Proben für den Nachweis von beispielsweise Drogen häufig Körperfluide zum Einsatz gelangen, welche große Mengen an Proteinen, Lipiden und Kohlenhydraten enthalten, die ihrerseits unerwünschte Folgen in immunchemischen Reaktionen hervorrufen können.

Erfindungsgemäß kann das Elutionsmittel auf beliebige Art und Weise in den ersten Bereich der mikrofluidischen Analysevorrichtung eingebracht werden. So kann das Elutionsmittel in einer Variante der Erfindung in dem erfindungsgemäß verwendeten Probennahmeelement gelagert sein. Zu diesem Zweck kann das Probennahmeelement beispielsweise eine das Elutionsmittel enthaltende Ampulle umfassen, welche bei Einbringen des Probennahmeelements in den ersten Bereich der mikrofluidischen Analysevorrichtung geöffnet wird und das Elutionsmittel freisetzt. Alternativ kann das Elutionsmittel in einem vierten Bereich der mikrofluidischen Analysevorrichtung gelagert sein, welcher mit dem ersten Bereich in fluider Kommunikation steht und aus welchem das Elutionsmittel, beispielsweise durch einen separat durchzuführenden Handhabungsschritt des Anwenders, in den ersten Bereich der mikrofluidischen Analysevorrichtung gespült werden kann (Fig. 1A und 1B).

Als Elutionsmittel kann im Rahmen der vorliegenden Erfindung grundsätzlich ein beliebiges Elutionsmittel verwendet werden, welches den Analyten aus dem Probennahmeelement herauszulösen vermag. Bevorzugt gelangen in dem hierin beschriebenen Verfahren allerdings wässrige Pufferlösungen zur Anwendung, welche gegebenenfalls weitere Reagenzien, insbesondere mindestens ein Protein, mindestens ein Kohlenhydrat, mindestens einen Zuckeralkohol, mindestens ein Detergenz oder/und mindestens ein organisches Lösungsmittel, wie jeweils oben beschrieben, in einer Konzentration von üblicherweise etwa 0.05 bis etwa 1.5 Gew.-% umfassen können. Als besonders bevorzugt wird im Rahmen des erfindungsgemäßen Verfahrens ein vorzugsweise wässriges Elutionsmittel angesehen, welches 3-[3-Cholamidopropyl)dimethyl-ammonio]-1-propansulfonat als Bestandteil umfasst. Durch geeignete Kombination obiger Reagenzien können in Abhängigkeit von der Struktur des jeweiligen Analyten Synergieeffekte erzielt werden, wodurch die Elution des Analyten aus dem Probennahmeelement bzw. das Herauslösen des Analyten aus der Probenmatrix verbessert und hierdurch eine Erhöhung der Sensitivität oder/und Spezifität der Analytbestimmung bewirkt werden kann.

Im Anschluss an das Eluieren des Analyten aus dem Probennahmeelement wird das erhaltene Eluat, d.h. das Gemisch aus analythaltiger Probe und Elutionsmittel, in einem weiteren Schritt des erfindungsgemäßen Verfahrens über geeignete Mittel, insbesondere über eine mikrofluidische Struktur wie beispielsweise einen Mikrokanal, in den zweiten Bereich der mikrofluidischen Analysevorrichtung überführt und mit einem in dem zweiten Bereich gelagerten analytspezifischen Bindungspartner für einen definierten Zeitraum in Kontakt gebracht, wobei eine Bindung des Analyten an den analytspezifischen Bindungspartner erfolgt Erfindungsgemäß wird der Analyt somit in Abwesenheit eines zum Nachweis des Analyten dienenden Testelements mit dem analytspezifischen Bindungspartner inkubiert, wodurch sowohl die Sensitivität als auch die Spezifität des Nachweisverfahrens, insbesondere bei der Bestimmung von schlecht wasserlöslichen Analyten, signifikant erhöht werden kann.

Die Überführung des Eluats aus dem ersten Bereich in den zweiten Bereich der mikrofluidischen Analysevorrichtung kann hierbei beispielsweise durch einen separat durchzuführenden Handhabungsschritt des Anwenders in Gang gesetzt werden, wobei vorzugsweise ein definiertes Volumen des Eluats in den zweiten Bereich transportiert wird. Die Überführung des Eluats aus dem ersten Bereich in den zweiten Bereich kann zudem genutzt werden, um einen noch höheren Grad an homogener Vermischung von Probe und Elutionsmittel zu erreichen. Dies kann vorzugsweise durch eine mikrofluidische Mischerstrecke oder eine andere dem Fachmann bekannte mikrofluidische Struktur erreicht werden.

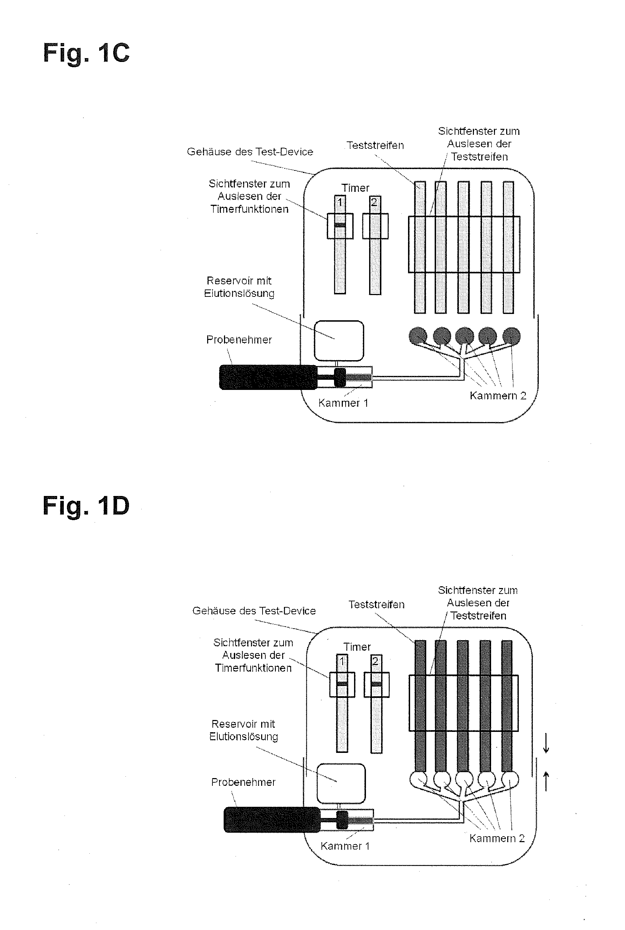

Der zweite Bereich der mikrofluidischen Analysevorrichtung kann grundsätzlich in beliebiger Form ausgestaltet sein, sofern er das aus dem ersten Bereich überführte Eluat aufnehmen kann und einen Kontakt des Eluats mit dem in dem zweiten Bereich vorgelegten analytspezifischen Bindungspartner ermöglicht. In einer bevorzugten Variante der Erfindung ist der zweite Bereich der mikrofluidischen Analysevorrichtung als einzelne Kammer ausgebildet und enthält einen einzelnen analytspezifischen Bindungspartner. Alternativ kann der zweite Bereich auch mehrere Kammern, beispielsweise 2 bis 5 Kammern, umfassen, welche vorzugsweise parallel zueinander angeordnet sind und gegebenenfalls unterschiedliche analytspezifische Bindungspartner enthalten können, sofern mehrere Analyten gleichzeitig bestimmt werden sollen (Fig. 1 C).

Der analytspezifische Bindungspartner kann eine beliebige chemische Substanz sein, welche an den zu testenden Analyten bindet, jedoch keinerlei Bindungen zu anderen in der Probe eventuell vorhandenen Stoffen ausbildet. Chemische Substanzen, welche diesem Anforderungsprofil genügen, sind dem Fachmann allgemein bekannt oder können entsprechend den Anforderungen an den jeweiligen Analyten mittels Routineexperimenten und unter Anwendung bekannter Techniken hergestellt werden.

Vorzugsweise handelt es sich bei dem analytspezifischen Bindungspartner um einen Antikörper oder ein funktionelles Antikörperfragment, welcher gegebenenfalls zusätzlich eine Bindungsstelle oder mehrere Bindungsstellen für ein Abfangreagenz, wie es oben beschrieben ist, aufweisen können. Der Begriff "funktionelles Antikörperfragment", wie er im Rahmen der vorliegenden Anmeldung verwendet wird, bezeichnet hierbei ein Antikörperfragment, das die ihm zugedachte Funktion einer spezifischen Bindung an den Analyten erfüllen kann. Ein "nicht-funktionelles Antikörperfragment" bezeichnet demgegenüber ein Antikörperfragment, welches keine Bindung oder keine spezifische Bindung mit dem Analyten ausbildet und insofern die ihm zugedachte Funktion einer spezifischen Bindung an den Analyten nicht erfüllt.

Um den Nachweis des Analyten zu erleichtern, kann der analytspezifische Bindungspartner gegebenenfalls eine detektierbare Markierung, wie beispielsweise eine Enzymmarkierung, Farbstoffmarkierung, Fluoreszenzmarkierung, Metallmarkierung oder Partikelmarkierung, umfassen. Für die Zwecke der vorliegenden Erfindung hat sich die Verwendung analytspezifischer Bindungspartner, welche eine optisch detektierbare Markierung, insbesondere eine Metallmarkierung, umfassen, als vorteilhaft erwiesen. Besonders bevorzugt handelt es sich bei der Markierung um eine Goldmarkierung, welche den Vorteil besitzt, dass das Testergebnis vom Anwender direkt optisch-visuell erfasst und ausgewertet werden kann. Techniken, mittels welcher oben beschriebene Markierungen in ein zu markierendes Molekül eingeführt werden können, sind dem Fachmann bekannt und werden insofern nicht näher erläutert.

Erfindungsgemäß kann der zweite Bereich den analytspezifischen Bindungspartner in jeder beliebigen Form enthalten, so lange bei Eintritt des Eluats in den zweiten Bereich der mikrofluidischen Analysevorrichtung eine Reaktion mit dem analytspezifischen Bindungspartner erfolgen kann. Vorzugsweise wird der analytspezifische Bindungspartner in dem zweiten Bereich der mikrofluidischen Analysevorrichtung indessen derart gelagert, dass er bei Einbringen des Eluats in den zweiten Bereich der mikrofluidischen Analysevorrichtung gelöst und homogen in der Lösung verteilt wird. Zu diesem Zweck kann die mikrofluidische Analysevorrichtung den analytspezifischen Bindungspartner beispielsweise in getrockneter Form enthalten, wobei grundsätzlich auch andere dem Fachmann geeignet erscheinende Lagerungsformen in Frage kommen.

Nach Inkontaktbringen und gegebenenfalls homogenem Vermischen von Eluat und analytspezifischem Bindungspartner wird üblicherweise eine immunologische Reaktion in Gang gesetzt, wobei ein Antigen-Antikörper-Komplex aus Analytmolekülen und analytspezifischem, gegebenenfalls markiertem Bindungspartner gebildet wird. Zur Wahrung einer hohen Sensitivität und Spezifität wird das Eluat mit dem darin enthaltenen Analyten für einen Zeitraum von mindestens 3 Sekunden, bevorzugt von mindestens 5 Sekunden mit dem analytspezifischen Bindungspartner in Kontakt gebracht, wobei eine Bindung des Analyten an den analytspezifischen Bindungspartner erfolgt. Als vorteilhaft hat sich in diesem Zusammenhang eine Reaktionszeit von etwa 5 Sekunden bis etwa 600 Sekunden, stärker bevorzugt von etwa 60 Sekunden bis etwa 300 Sekunden, am stärksten bevorzugt von etwa 90 Sekunden bis etwa 240 Sekunden, erwiesen.

Die Reaktion zwischen dem Analyten und dem analytspezifischen Bindungspartner kann grundsätzlich auf beliebige Art und Weise erfolgen, sofern ein ausreichender Kontakt zwischen dem Analyten und dem analytspezifischen Bindungspartner gewährleistet ist. Für die Zwecke der vorliegenden Erfindung hat es sich indessen als vorteilhaft erwiesen, wenn die Reaktion zwischen dem Analyten und dem analytspezifischen Bindungspartner in stationärer Phase durchgeführt wird, so dass eine ausreichend lange Kontaktzeit zur Bildung eines Komplexes aus Analyt und analytspezifischem Bindungspartner zur Verfügung steht.

Um die Reaktion zwischen dem Analyten und dem analytspezifischen Bindungspartner zu begünstigen, kann der zweite Bereich der mikrofluidischen Analysevorrichtung bei Bedarf weitere Reagenzien umfassen. Als vorteilhaft gelten in diesem Zusammenhang insbesondere Reagenzien, welche ein Auflösen des analytspezifischen Bindungspartners bei Kontakt mit dem Eluat fördern, eine vollständige Vermischung von Eluat und analytspezifischem Bindungspartner unterstützen, bzw. eine optimale Reaktion zwischen dem Analyten und dem analytspezifischen Bindungspartner sicherstellen. Reagenzien, welche zu diesem Zweck eingesetzt werden können, umfassen beispielsweise Proteine, Kohlenhydrate, Zuckeralkohole, Detergenzien oder/und Salze, wie sie oben definiert sind.

In einem weiteren Schritt des erfindungsgemäßen Verfahrens wird das in dem zweiten Bereich der mikrofluidischen Analysevorrichtung erhaltene Reaktionsgemisch, d.h. das Gemisch aus analythaltiger Probe, Elutionsmittel und analytspezifischem Bindungspartner, über geeignete Mittel, wie beispielsweise einen Mikrokanal, in den dritten Bereich der mikrofluidischen Analysevorrichtung überführt und dort mit mindestens einem Testelement in Kontakt gebracht, auf welchem eine nachfolgende Bestimmung des Analyten erfolgen kann (Fig. 1D). Die Überführung des Reaktionsgemisches aus dem zweiten Bereich in den dritten Bereich kann beispielsweise durch einen separat durchzuführenden Handhabungsschritt des Anwenders, beispielsweise durch Öffnen eines Mikrokanals, in Gang gesetzt werden, wobei vorzugsweise ein definiertes Volumen des zu transportierenden Gemisches in den dritten Bereich der mikrofluidischen Analysevorrichtung überführt wird und, beispielsweise unter Nutzung von Kapillareffekten, auf das mindestens eine Testelement gelangt. In einer bevorzugten Variante der Erfindung umfasst der dritte Bereich mehr als ein Testelement, beispielsweise 2 bis 5 Testelemente, welche alsdann vorzugsweise parallel zueinander angeordnet sind.

Die erfindungsgemäß verwendeten Testelemente umfassen grundsätzlich jede dem Fachmann geläufige physikalische Form, welche für die Bestimmung des Vorhandenseins oder/und der Menge eines Analyten in einer Probe geeignet ist. Hierbei ist das Testelement vorzugsweise derart ausgebildet, dass es bei Anwesenheit des zu bestimmenden Analyten ein optisch detektierbares Signal erzeugt, welches eine qualitative oder/und quantitative Bestimmung des Analyten ermöglicht. Beispiele für Testelemente im Sinne der vorliegenden Erfindung umfassen u.a. Teststreifen, Testbänder und Testpads, auf die der Analyt beispielsweise in Form einer wässrigen oder nichtwässrigen Lösung aufgebracht werden kann.

Bevorzugt handelt es sich bei dem Testelement um einen chromatographischen Teststreifen, welcher in einer Variante der Erfindung aus einem einzigen chromatographiefähigen, gegebenenfalls streifenförmigen Material gebildet sein kann. Vorzugsweise umfasst der chromatographische Teststreifen allerdings mehrere, auf einer Trägerschicht überlappend angeordnete kapillaraktive Flächen aus gleichen oder unterschiedlichen chromatographischen Materialien, welche in fluider Kommunikation miteinander stehen und auf diese Weise eine Transportstrecke bilden, entlang derer ein Fluid, durch Kapillarkräfte getrieben, durch sämtliche Bereiche des Testelements strömen kann. Als chromatographisches Material kann dabei jedes bekannte flüssigkeitsabsorbierende, poröse oder kapillaraktive Material verwendet werden, wie z.B. Cellulose und Derivate hiervon, Glasfasern, sowie Vliese und Gewebe aus künstlichen oder natürlichen Materialien. Chromatographische Teststreifen, welche im Rahmen der vorliegenden Erfindung Anwendung finden können, sind beispielsweise in EP 0 699 906 A2 beschrieben, auf deren Offenbarung hiermit ausdrücklich Bezug genommen wird.

Um eine Bestimmung des Analyten, beispielsweise auf Basis eines kompetitiven Testformats oder eines Sandwich-Testformats, zu ermöglichen, können die Testelemente mehrere voneinander räumlich abgrenzbare Zonen umfassen, welche vorzugsweise eine unterschiedliche Funktion erfüllen und, sofern erforderlich, mit unterschiedlichen Reagenzien bestückt sind. Besonders bevorzugt ist im Rahmen des erfindungsgemäßen Verfahrens die Verwendung eines Testelements, welches (a) eine erste Zone, die zur Aufnahme eines Laufmittels ausgebildet ist, (b) eine zweite Zone, die eine markierte Kontrollsubstanz umfasst, (c) eine dritte Zone, die zur optischen Detektion des Analyten ausgebildet ist, und (d) eine vierte Zone, die zur Aufnahme von überschüssigem Laufmittel ausgebildet ist, umfasst.

Vorzugsweise umfasst das Testelement eine endständige Zone, welche zur Aufnahme von Laufmittel ausgebildet ist und üblicherweise ein saugfähiges Material, wie beispielsweise Gewebe oder/und Vlies, umfasst. Nach Benetzung dieser Zone mit Laufmittel, bei welchem es sich im Rahmen des erfindungsgemäßen Verfahrens um das in dem zweiten Bereich der mikrofluidischen Analysevorrichtung erhaltene Reaktionsgemisch aus analythaltiger Probe, Elutionsmittel und analytspezifischem Bindungspartner handelt, wandert das Laufmittel durch die verschiedenen Zonen des Testelements. Hierbei kann vorteilhafterweise die Kapillarwirkung der einzelnen Komponenten des Testelements ausgenutzt werden, welche derart angeordnet bzw. miteinander verbunden sind, dass ein ununterbrochener Laufmittelfluss gewährleistet ist.

Die zur optischen Detektion des Analyten ausgebildete Zone umfasst üblicherweise mehrere definierte Abschnitte, in welchen unterschiedliche Reagenzien immobilisiert sein können. Bevorzugt umfasst diese Zone insofern (a) einen Abschnitt, welcher zur Bindung von ungebundenem analytspezifischem Bindungspartner ausgebildet ist, (b) einen Abschnitt, welcher zur Bindung des Komplexes aus Analyt und analytspezifischem Bindungspartner ausgebildet ist, und (c) einen Abschnitt, in welchem Analyten-unabhängig ein Kontrollsignal erzeugt wird. Die zur optischen Detektion des Analyten ausgebildete Zone kann dabei aus einem oder mehreren Materialien gebildet sein, welche dem Fachmann für die Zwecke der Erfindung geeignet erscheinen, wie beispielsweise Membranen aus Nylon^{®}, Nitrocellulose oder Polyvinylidenfluorid.

Der zur Bindung von ungebundenem analytspezifischem Bindungspartner vorgesehene Abschnitt kann beispielsweise immobilisierte Analytanaloga, insbesondere Polyhaptene, umfassen, welche den analytspezifischen, gegebenenfalls markierten Bindungspartner infolge Ausbildung eines Komplexes an einer definierten Position auf dem Testelement (Abfanglinie) abfangen und auf diese Weise die Erzeugung falsch-positiver Ergebnisse vermeiden. Der Komplex aus Analyt und analytspezifischem Bindungspartner wird an der Abfanglinie üblicherweise nicht immobilisiert, da der Analyt die hierzu erforderlichen Bindungsstellen am analytspezifischen Bindungspartner blockiert.

Der zur Bindung des Komplexes aus Analyt und analytspezifischem Bindungspartner ausgebildete Abschnitt umfasst vorzugsweise einen für den analytspezifischen Bindungspartner spezifischen Bindungspartner, welcher eine Immobilisierung des Komplexes aus Analyt und analytspezifischem Bindungspartner an einer vorbestimmten Position auf dem Testelement (Testlinie) bewirkt und auf diese Weise eine optische Bestimmung des Analyten ermöglicht. Die Immobilisierung des Komplexes erfolgt in der Regel über eine freie Bindungsstelle des analytspezifischen Bindungspartners, wobei eine Färbung der Testlinie mit einem positiven Nachweis des Analyten einhergeht.

Um das Signal an der Testlinie eindeutig ablesen zu können und Verwechslungen mit der Abfanglinie auszuschließen, kann die Abfanglinie gegebenenfalls in geeigneter Weise abgedeckt werden. Der Kontrollabschnitt umfasst vorzugsweise einen für die Kontrollsubstanz aus der zweiten Zone des Testelements geeigneten Bindungspartner in immobilisierter Form, wodurch die Kontrollsubstanz bei Kontakt mit ihrem Bindungspartner immobilisiert wird und an einer vorbestimmten Position auf dem Testelement (Kontrolllinie) ein detektierbares Signal erzeugt. Die Kontrolllinie wird unabhängig von der Anwesenheit des Analyten ausgebildet wird und fungiert als Indikator für eine einwandfreie Funktionalität des Testelements. Überschüssiges Laufmittel, welches die zur optischen Detektion des Analyten ausgebildete Zone des Testelements verlässt, kann mit Hilfe eines flüssigkeitsabsorbierenden Materials in einer eigens hierfür ausgebildeten Zone des Testelements aufgenommen werden.

Die im letzten Schritt des erfindungsgemäßen Verfahrens durchgeführte qualitative oder/und quantitative Bestimmung des Analyten kann auf beliebige Art und Weise erfolgen. Hierzu können grundsätzlich alle aus dem Stand der Technik bekannten Methoden zum Nachweis immunologischer Reaktionen eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, insbesondere photometrische oder fluorimetrische Nachweismethoden, zum Einsatz. Besonders bevorzugt ist erfindungsgemäß ein optisch-visueller Nachweis des Analyten.

In einer bevorzugten Variante des erfindungsgemäßen Verfahrens umfasst die mikrofluidische Analysevorrichtung neben oben genannten Bereichen einen weiteren Bereich, welcher mindestens eine Timerfunktion zur zeitlichen Überwachung der in der Analysevorrichtung ablaufenden Vorgänge bzw. Reaktionen umfasst. Der Begriff "Timerfunktion", wie er im Rahmen der vorliegenden Anmeldung verwendet wird, bezeichnet einen Zeitmesser, welcher dem Anwender, beispielsweise durch ein optisches oder/und akustisches Signal, das Ende eines in der Analysevorrichtung ablaufenden Vorgangs, insbesondere das Ende einer in der Analysevorrichtung ablaufenden Reaktion, anzeigt und gegebenenfalls einen weiteren Handhabungsschritt des Anwenders, wie beispielsweise das Drücken eines Knopfes an der Analysevorrichtung zum Auslesen des Testergebnisses, erforderlich machen kann.

Besonders bevorzugt wird mittels oben genannten Zeitmessers die Dauer der Reaktion zwischen dem Analyten und dem analytspezifischen Bindungspartner im zweiten Bereich der mikrofluidischen Analysevorrichtung [Schritt (d)] oder/und die Dauer der Analytbestimmung auf dem Testelement im dritten Bereich der mikrofluidischen Analysevorrichtung [Schritt (f)] überwacht, wobei die mikrofluidische Analysevorrichtung für jeden der beiden Schritte vorzugsweise einen eigenen Zeitmesser vorsieht und insofern vorzugsweise zwei voneinander unabhängige Zeitmesser umfasst.

Was Schritt (d) des erfindungsgemäßen Verfahrens anbetrifft, so startet der Zeitmesser üblicherweise bei Einbringen des Eluats, d.h. des Gemisches aus analythaltiger Probe und Elutionsmittel, in den zweiten Bereich der mikrofluidischen Analysevorrichtung. Dieser erste Zeitmesser zeigt dem Anwender an, dass die Reaktion zwischen dem Analyten und dem analytspezifischen Bindungspartner beendet ist und damit die Überführung des Gemisches aus dem zweiten in den dritten Bereich der mikrofluidischen Analysevorrichtung initialisiert werden kann.

In Bezug auf Schritt (f) startet der Zeitmesser üblicherweise mit der Überführung des Reaktionsgemisches aus analythaltiger Probe, Elutionsmittel und analytspezifischem Bindungspartner in den dritten Bereich der mikrofluidischen Analysevorrichtung. Dieser zweite Zeitmesser ist derart eingestellt, dass sie mit dem Dauer der Analytbestimmung auf dem Testelement korreliert, und zeigt dem Anwender an, wann er das Testergebnis auslesen kann. Insofern können falsche Testergebnisse aufgrund zu früher Auslesung des Testelements sowie unnötige Verzögerungen bei der Auswertung aufgrund zu langer Wartezeiten vermieden werden.

Ein definierter und konstanter Zeitmesser kann u.a. durch eine Chromatographie dargestellt werden, welche zeitgleich zu den in Schritt (d) oder/und Schritt (f) ablaufenden Reaktionen durchgeführt wird. Hierzu kann beispielsweise ein Lateral-Flow-Teststreifen verwendet werden, welcher aus einer oder mehreren nebeneinander angeordneten kapillaraktiven Flächen besteht, die in fluider Kommunikation stehen und einen kontinuierlichen Flüssigkeitstransport ermöglichen. In einer bevorzugten Variante umfasst der Teststreifen mindestens einen Farbstoff als optischen Indikator, welcher an unterschiedlichen Positionen auf dem Teststreifen aufgebracht sein und, aufgrund der Abhängigkeit der Chromatographiedauer vom Laufmittel, nach einem definierten Zeitraum, beispielsweise durch Farbänderung oder/und durch Erscheinen in einem Auslesefenster, detektiert werden kann.

Sofern die mikrofluidische Analysevorrichtung mindestens einen Zeitmesser zur zeitlichen Überwachung der in der Analysevorrichtung ablaufenden Reaktionen umfasst, so beinhaltet sie vorzugsweise zusätzlich Mittel zum Initiieren bzw. Durchführen des Zeitmessers, wie beispielsweise mindestens ein für chromatographische Anwendungen geeignetes Laufmittel. Diese Mittel zum Initiieren bzw. Durchführen des Zeitmessers können in einem der oben beschriebenen Bereiche der mikrofluidischen Analysevorrichtung oder aber in einem hiervon separaten, weiteren Bereich gelagert sein, wobei letztere Variante als bevorzugt gilt. Alternative Ausgestaltungen der mikrofluidischen Analysevorrichtung bzw. deren einzelner Bereiche ergeben sich für den Fachmann auf Grundlage seines allgemeinen Fachwissens in Kombination mit obigen Ausführungen.

Das erfindungsgemäße Verfahren ermöglicht die Bestimmung eines oder mehrerer Analyten mit hoher Sensitivität und Spezifität. So ist es erfindungsgemäß bevorzugt, Analyten mit einer Spezifität von mindestens 95% oder/und einer Sensitivität von mindestens 90% zu bestimmen. Stärker bevorzugt erfolgt die Bestimmung mit einer Spezifität von mindestens 98% oder/und einer Sensitivität von mindestens 95%, so dass mittels des hierin beschriebenen Verfahrens Analyten bis hinab zu einer unteren Nachweisgrenze von etwa 1 ng/ml Probe nachgewiesen werden können.

Das erfindungsgemäße Verfahren kann zur Bestimmung einer beliebigen biologischen oder chemischen Substanz verwendet werden, welche immunologisch nachweisbar ist. Vorzugsweise wird das hierin beschriebene Verfahren indessen zum Nachweis eines Analyten ausgewählt aus der Gruppe bestehend aus Amphetaminen, Methamphetaminen, Cannabinoiden, insbesondere Δ⁹-Tetrahydrocannabinol, Opiaten, insbesondere Morphin, Codein oder Dihydrocodein, Opioiden, insbesondere Heroin, Tropan-Alkaloiden, insbesondere Kokain, oder Benzodiazepinen verwendet, wobei Δ⁹-Tetrahydrocannabinol und Kokain als Analyten besonders bevorzugt sind.

Der Analyt kann aus einer beliebigen Quelle stammen, wie beispielsweise von einer Oberfläche eines mit dem Analyten benetzten Gegenstandes oder aus einem Körperfluid, wie beispielsweise Vollblut, Plasma, Serum, Urin, Speichel oder Schweiß. Bevorzugt wird mittels des hierin beschriebenen Verfahrens das Vorhandensein oder/und die Menge eines Analyten in einer Probe aus Vollblut, Urin oder Speichel bestimmt. Die zur Durchführung des Verfahrens benötigte Probenmenge beträgt üblicherweise von etwa 5 µl bis etwa 500 µl, bevorzugt von etwa 10 µl bis etwa 250 µl, und am stärksten bevorzugt von etwa 30 µl bis etwa 150 µl.

In einem weiteren Aspekt betrifft die Erfindung einen Kit, welcher vorzugsweise zur Durchführung des vorstehend beschriebenen Verfahrens verwendet wird und nachfolgende Bestandteile umfasst:
(a) eine mikrofluidische Analysevorrichtung zur Bestimmung eines Analyten, umfassend:
   (i) einen ersten Bereich, welcher zum Einbringen eines Probennahmeelements mit hierauf aufgenommenem Analyten und zum Eluieren des Analyten von dem Probennahmeelement ausgebildet ist,
   (ii) einen zweiten Bereich, welcher einen für den Analyten spezifischen Bindungspartner enthält und mit dem ersten Bereich durch Mikrokanäle, Stufen, Verzweigungen oder/und Kammern verbunden ist, wobei der zweite Bereich kein zum Nachweis des Analyten dienendes Testelement umfasst,
   (iii) einen dritten Bereich, welcher mindestens ein Testelement zur Bestimmung des Vorhandenseins oder/und der Menge des Analyten umfasst und mit dem zweiten Bereich durch Mikrokanäle, Stufen, Verzweigungen oder/und Kammern verbunden ist,
   (iv) gegebenenfalls einen vierten Bereich, welcher ein Elutionsmittel zum Eluieren des Analyten von dem Probennahmeelement umfasst und mit dem ersten Bereich in fluider Kommunikation steht,
   (v) gegebenenfalls einen fünften Bereich, welcher mindestens eine Timerfunktion zur zeitlichen Überwachung der in der Analysevorrichtung ablaufenden Reaktionen umfasst, und
   (vi) gegebenenfalls ein Gehäuse, und
(b) ein Probennahmeelement, welches in die mikrofluidische Analysevorrichtung eingebracht werden kann und gegebenenfalls ein Elutionsmittel zum Eluieren des Analyten von dem Probennahmeelement umfasst.

Was bevorzugte Ausgestaltungen der in obigem Kit enthaltenen mikrofluidischen Arialysevorrichtung, des Probennahmeelements sowie des Testelements anbetrifft, so wird auf die Ausführungen in Zusammenhang mit der Beschreibung des erfindungsgemäßen Verfahrens verwiesen.

Die Erfindung soll durch die nachfolgenden Figuren und Beispiele näher erläutert werden:

### Beschreibung der Figuren

- **Figur 1:**: Darstellung einer Ausführungsform einer mikrofluidischen Analysevorrichtung zur Durchführung des Verfahrens gemäß der vorliegenden Erfindung, umfassend:
(a) eine erste Kammer zum Einbringen eines Probennahmeelements und zum Eluieren von Analyt aus dem Probennahmeelement,
(b) fünf zweite Kammern, welche jeweils einen analytspezifischen Bindungspartner enthalten und mit der ersten Kammer in fluider Kommunikation stehen,
(c) fünf Teststreifen zur Bestimmung des Vorhandenseins oder/und der Menge an Analyt, welche jeweils mit einer der zweiten Kammern in fluider Kommunikation stehen,
(d) ein Reservoir mit Elutionsmittel, welches mit der ersten Kammer in fluider Kommunikation steht, und
(e) zwei Timer zur zeitlichen Überwachung der in der Analysevorrichtung ablaufenden Reaktionen.
   **1A:** Einbringen eines Probennahmeelements in die erste Kammer der mikrofluidischen Analysevorrichtung,
   **1B:** Eluieren von Analyt aus dem Probennahmeelement in der ersten Kammer mittels des in dem Reservoir enthaltenen Elutionsmittels,
   **1C:** Überführen des Eluats in die zweiten Kammern und Inkontaktbringen mit dem analytspezifischen Bindungspartner bzw. den analytspezifischen Bindungspartnern,
   **1D:** Überführen des Reaktionsgemisches auf die Teststreifen und Bestimmen des/der Analyten.

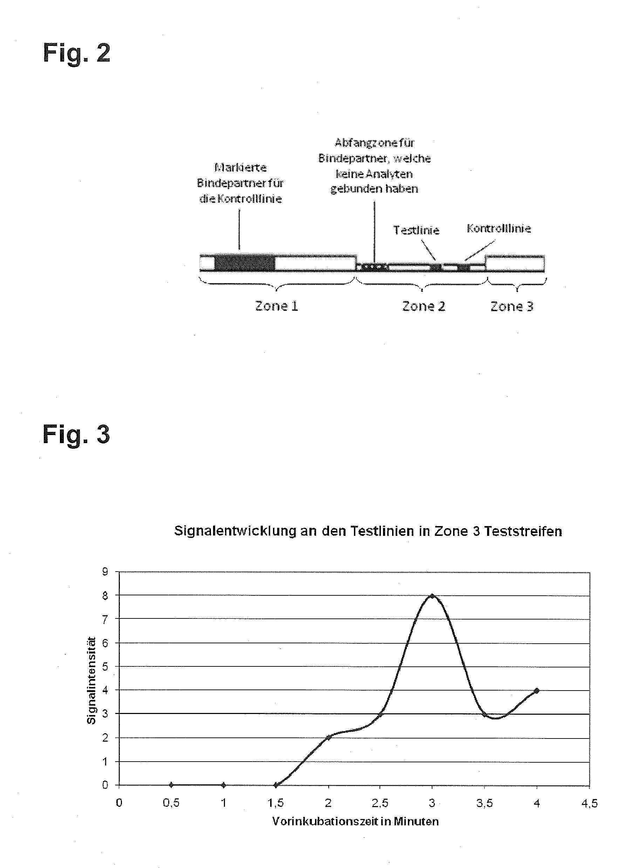

- **Figur 2:**: Querschnitt einer Ausführungsform eines Testelements zur Durchführung des Verfahrens gemäß der vorliegenden Erfindung. Das in Form eines Teststreifens dargestellte Testelement umfasst:
(a) eine erste Zone, die eine markierte Kontrollsubstanz sowie gegebenenfalls weitere Reagenzien umfasst,
(b) eine zweite Zone, die zur optischen Detektion des Analyten ausgebildet ist und eine Abfanglinie, eine Testlinie und eine Kontrolllinie umfasst, und
(c) eine dritte Zone, die zur Aufnahme von überschüssigem Laufmittel ausgebildet ist.
- **Figur 3:**: Abhängigkeit der Sensitivität der Bestimmung von Δ⁹-THC von der Dauer der Reaktion zwischen Analyt und analytspezifischem Bindungspartner
- **Figur 4:**: Darstellung verschiedener Teststreifen mit hierauf aufgebrachtem Farbstoff, welche die Festlegung definierter und konstanter Zeiträume ermöglichen.
4A: Teststreifen mit immobilisierten Farbstoff, welcher nach einem definierten Zeitraum seine Farbe ändert,
**4B:** Teststreifen mit Farbstoff, welcher nach einem definierten Zeitraum in einem Auslesefenster erscheint.

### Beispiele

### Beispiel 1: Herstellung von Δ⁹-THC-lmmunogen

1.25 ml einer Stammlösung von Δ⁹-Tetrahydrocannabinolsäure A (1-Hydroxy-(-)-(6aR,10aR)-6,6,9-trimethyl-3-pentyl-6a,7,8,10a-tetrahydrobenzo[c]chromen-2-carbonsäure; Firma Lipomed) in N,N-Dimethylformamid (Konzentration: 100.8 mg/ml) wurden mit 8.75 ml N,N-Dimethylformamid auf ein Endvolumen von 10 ml verdünnt (Endkonzentration: 12.6 mg/ml).

5 ml dieser verdünnten Lösung wurden anschließend unter Rühren zu einer wässrigen Lösung von Rinderserumalbumin (BSA), welche vorab durch Lösen von 250 mg Rinderserumalbumin (Firma Sigma Aldrich) in einem Gemisch aus 25 ml destilliertem Wasser und 2.5 ml N,N-Dimethylformamid hergestellt worden war, hinzugefügt. Nach Zugabe von 6.3 ml destilliertem Wasser zu dem erhaltenen Reaktionsgemisch wurden aufeinander folgend die restlichen 5 ml der oben beschriebenen verdünnten Lösung von Δ⁹-Tetrahydrocannabinolsäure A in N,N-Dimethylformamid, weitere 6 ml destilliertes Wasser, sowie 0.2 ml einer Lösung von 1-Ethyl-3-(3-dimethylaminopropyl)carbodiimid-Hydrochlorid (Firma Sigma Aldrich) in N,N-Dimethylformamid (Konzentration 500 mg/ml) zugesetzt.

Das Becherglas mit der darin enthaltenen Lösung wurde in Aluminiumfolie eingewickelt und auf einem Magnetrührer für 24 h bei 5°C gerührt. Anschließend wurde die Lösung in einen Dialysebehälter überführt und über einen Zeitraum von 4 Tagen bei 5°C gegen eine 25% Lösung von N,N-Dimethylformamid in Wasser dialysiert. Das Dialysat wurde mittels Immunoelektrophorese getestet. Inkubation mit einem Ziege-anti-BSA-Antikörper (Firma Genetex) sowie eine Präzipitatbande zeigten, dass sich das Δ⁹-THC-lmmunogen von der Bande des Rinderserumalbumins unterschied.

### Beispiel 2: Gewinnung von Antikörpern gegen Δ⁹-THC

20 Mäuse wurden mit dem in Beispiel 1 erhaltenen Δ⁹-THC-lmmunogen in komplettem Freund-Adjuvans (Firma Sigma Aldrich) immunisiert, wobei die Dosis für die erste und jede weitere Immunisierung jeweils 75 µg Immunogen pro Tier betrug. Die Immunisierungen erfolgten über einen Zeitraum von 6 Monaten jeweils in einem Abstand von einem Monat.

Die aus den immunisierten Mäusen erhaltenen Seren wurden anschließend in einem Mikrotiterplatten-Assay auf die Anwesenheit von Antikörpern gegen Δ⁹-THC untersucht. Hierzu wurden mit Streptavidin beschichtete Mikrotiterplatten zunächst mit 10 ml einer Lösung von Δ⁹-Tetrahydrocannabinol-[N'-biotinylaminocaproyl-(3,6-dioxa-8-aminooctyl)-amid] in phosphatgepufferter Salzlösung, das vorab durch Umsetzung von Δ⁹-Tetrahydrocannabinolsuccinimidylester und N-(Biotinylaminocaproyl)-1,8-diamino-3,6-dioxaoctan (Firma Applichem) hergestellt worden war, für 12 h bei 4°C inkubiert.

Nach Waschen mit 20 ml einer 0.05% Lösung von Tween 20 in phosphatgepufferter Salzlösung wurden die Mikrotiterplatten jeweils für 1 h bei 20°C mit 10 ml der zu untersuchenden Seren inkubiert, und anschließend erneut mit 20 ml einer 0.05% Lösung von Tween 20 in phosphatgepufferter Salzlösung gewaschen. Zur Detektion wurde für 1 h bei 20°C mit 10 ml einer Lösung eines Konjugats aus Peroxidase und Kaninchen-anti-Maus-IgG (Firma Dako) inkubiert, mit 20 ml einer 0.05% Lösung von Tween 20 in phosphatgepufferter Salzlösung gewaschen, und mit Substrat versetzt. Seren mit einer guten Affinität gegenüber Δ⁹-THC wurden zur Gewinnnung von Antikörpern gegen Δ⁹-THC ausgewählt.

### Beispiel 3: Herstellung eines Gold-markierten Antikörpers gegen Δ⁹-THC

Goldsol mit einem durch Photonen-Korrelations-Spektroskopie bestimmten Partikeldurchmesser von 20 nm wurde in Anlehnung an den Stand der Technik (Frens, Nature (1973), 241, 20-22) hergestellt. Die Markierung der in Beispiel2 gewonnenen, Δ⁹-THC-spezifischen Antikörper mit den Goldpartikeln erfolgte in Übereinstimmung mit bekannten Verfahren (Geoghegan et al., J. Immunol. Meth. (1980), 34, 11-31).

### Beispiel 4: Einfluss von 3-[3-Cholamidopropyl)dimethylammonio]-1-propansulfonat (CHAPS) im Elutionsmittel auf die Sensitivität

Poolspeichel (Speichelmix von fünf Personen) wurde mit einer in Methanol verdünnten Lösung von Δ⁹-THC (Firma Cerilliant) auf eine Δ⁹-THC-Konzentration von 50 ng/ml eingestellt. Alsdann wurden 100 µl dieses dotierten Poolspeichels mit einem handelsüblichen Probennahmeelement (Firma CopanFlock Technologies) aufgenommen und das Probennahmeelement in die erste Kammer einer mikrofluidischen Analysevorrichtung, wie sie in Fig. 1 dargestellt ist, eingebracht.

Anschließend wurde das Probennahmeelement in der ersten Kammer der mikrofluidischen Analysevorrichtung für 20 Sekunden mit vier unterschiedlich konzentrierten Lösungen von 3-[3-Cholamidopropyl)-dimethylammonio]-1-propansulfonat (Firma Sigma Aldrich) in destilliertem Wasser versetzt, wobei die analythaltige Probe aus dem Probennahmeelement eluiert und homogen im Elutionsmittel verteilt wurde.

Das Gemisch aus analythaltiger Probe und Elutionsmittel wurde alsdann über ein mikrofluidisches Kapillarsystem in eine zweite Kammer der mikrofluidischen Analysevorrichtung, welche einen Δ⁹-THC spezifischen Antikörper gemäß Beispiel 3 enthielt, überführt und mit dem analytspezifischen Bindungspartner im stationären Zustand für einen Zeitraum von 120 bis 180 Sekunden in Kontakt gebracht.

Die Geometrie der zweiten Kammer war mit einer Länge von 3.2 cm, einer Breite von 1 cm, und einer Höhe von 2.1 mm derart ausgelegt, dass eine große Diffusionsfläche zwischen dem Eluat und dem auf der Bodenfläche der Kammer in getrockneter Form vorliegenden analytspezifischen Bindungspartner (eingetrocknete Lösung des Δ⁹-THC spezifischen Antikörpers in 50 mM Carbonatpuffer, pH 6) zustande kam. Mikroskopische Untersuchungen zeigten, dass nach Inkubation der analythaltigen Probe mit dem analytspezifischen Bindungspartner keine Rückstände des getrockneten analytspezifischen Bindungspartners in der zweiten Kammer vorlagen.

Nach Beendigung der stationären Phase wurde das Reaktionsgemisch über ein mikrofluidisches Kapillarsystem auf einen Teststreifen überführt und der Chromatographieprozess hierdurch gestartet. Die vollständige Testentwicklung betrug 5 bis 10 Minuten. Die Ergebnisse der vier Bestimmungen sind in Tabelle 1 dargestellt:

**Tabelle 1**

| CHAPS-Konzentration im Elutionsmittel (Gew.-%) | 0% | 0.01% | 0.1% | 1.0% |
|---|---|---|---|---|
| Signalintensität für Δ⁹-THC | 0 | 1 | 6 | 3 |

Wie aus Tabelle 1 hervorgeht, führt die Elution mit unterschiedlich konzentrierten Lösungen von CHAPS in Wasser zu einer unterschiedlichen Signalentwicklung an der Testlinie des chromatographischen Teststreifens. Die Skalierung der Signalintensität umfasst 9 Einheiten, wobei Signale im Bereich von 0-3 als negativ, Signale im Bereich von 4-5 als schwach positiv, und Signale im Bereich von > 6 als positiv anzusehen sind. Im einzelnen zeigt sich, dass ab einer Konzentration von 0.01 Gew.-% an CHAPS im Elutionsmittel eine Zunahme der Sensitivität der Analytbestimmung eintritt. Die beste Sensitivität lässt sich mit einer Konzentration von 0.1 Gew.-% an CHAPS im Elutionsmittel erzielen.

### Beispiel 5: Einfluss der Durchmischung von Analyt und Elutionsmittel auf die Sensitivität

Poolspeichel (Speichelmix von fünf Personen) wurde mit einer in Methanol verdünnten Lösung von Δ⁹-THC (Firma Cerilliant) auf eine Δ⁹-THC-Konzentration von 200 ng/ml eingestellt. Alsdann wurden 300 µl dieses dotierten Poolspeichels mit einem handelsüblichen Probennahmeelement (Firma CopanFlock Technologies) aufgenommen und das Probennahmeelement, in die erste Kammer einer mikrofluidischen Analysevorrichtung, wie sie in Fig. 1 dargestellt ist, eingebracht.

Anschließend wurde das Probennahmeelement in der ersten Kammer der mikrofluidischen Analysevorrichtung für 40 Sekunden mit 900 µl Elutionsmittel (25 mM Tris mit 0.5% Cholat) versetzt, wobei die analythaltige Probe aus dem Probennahmeelement eluiert wurde. Anschließend wurde in der ersten Kammer unter Verwendung einer mikrofluidischen Mischerstruktur ein homogenes Gemisch aus analythaltiger Probe und Elutionsmittel gebildet; in einem Vergleichsversuch wurde von einer homogenen Verteilung des Analyten im Elutionsmittel abgesehen.

Das Gemisch aus analythaltiger Probe und Elutionsmittel wurde alsdann in eine zweite Kammer, welche einen Δ⁹-THC spezifischen Antikörper gemäß Beispiel 3 enthielt, überführt und mit dem analytspezifischen Bindungspartner im stationären Zustand für einen Zeitraum von 120 bis 180 Sekunden in Kontakt gebracht.

Die Geometrie der zweiten Kammer war mit einer Länge von 3.2 cm, einer Breite von 1 cm, und einer Höhe von 2.1 mm derart ausgelegt, dass eine große Diffusionsfläche zwischen dem Eluat und dem auf der Bodenfläche der Kammer in getrockneter Form vorliegenden analytspezifischen Bindungspartner (eingetrocknete Lösung des Δ⁹-THC spezifischen Antikörpers in 50 mM Carbonatpuffer, pH 6) zustande kam. Mikroskopische Untersuchungen zeigten, dass nach Inkubation der analythaltigen Probe mit dem analytspezifischen Bindungspartner keine Rückstände des getrockneten analytspezifischen Bindungspartners in der zweiten Kammer vorlagen.

Nach Beendigung der stationären Phase wurde das Reaktionsgemisch über ein mikrofluidisches Kapillarsystem auf einen Teststreifen überführt und der Chromatographieprozess hierdurch gestartet. Die vollständige Testentwicklung betrug 5 bis 10 Minuten. Hierbei zeigte sich, dass die unterschiedliche Durchmischung von analythaltiger Probe und Elutionsmittel in der ersten Kammer der mikrofluidischen Analysevorrichtung zu einer unterschiedlich starken Signalentwicklung an der Testlinie des chromatographischen Teststreifens führt.

Die Skalierung der Signalintensität umfasst 9 Einheiten, wobei Signale im Bereich von 0-3 als negativ, Signale im Bereich von 4-5 als schwach positiv, und Signale im Bereich von > 6 als positiv anzusehen sind. Der Ansatz mit dem homogenen Gemisch aus analythaltiger Probe und Elutionsmittel zeigte eine Signalintensität von sieben Einheiten, während der nicht-durchmischte Vergleichsansatz eine signifikant schwächere Signalintensität von lediglich zwei Einheiten ergab.

### Beispiel 6: Einfluss der Inkubationszeit von Analyt und analytspezifischem Bindungspartner auf die Sensitivität

Poolspeichel (Speichelmix von fünf Personen) wurde mit einer in Methanol verdünnten Lösung von Δ⁹-THC (Firma Cerilliant) auf eine Δ⁹-THC-Konzentration von 50 ng/ml eingestellt. Alsdann wurden 100 µl dieses dotierten Poolspeichels mit einem handelsüblichen Probennahmeelement (Firma CopanFlock Technologies) aufgenommen und das Probennahmeelement in die erste Kammer einer mikrofluidischen Analysevorrichtung, wie sie in Fig. 1 dargestellt ist, eingebracht.

Anschließend wurde das Probennahmeelement in der ersten Kammer der mikrofluidischen Analysevorrichtung für 20 Sekunden mit Elutionsmittel (destilliertes Wasser) versetzt, wobei die analythaltige Probe aus dem Probennahmeelement eluiert und homogen im Elutionsmittel verteilt wurde.

Das Gemisch aus analythaltiger Probe und Elutionsmittel wurde alsdann über ein mikrofluidisches Kapillarsystem in eine zweite Kammer der mikrofluidischen Analysevorrichtung, welche einen Δ⁹-THC spezifischen Antikörper gemäß Beispiel 3 enthielt, überführt und mit dem analytspezifischen Bindungspartner im stationären Zustand für unterschiedlich lange Zeiträume in Kontakt gebracht (Inkubationszeit).

Die Geometrie der zweiten Kammer war mit einer Länge von 3.2 cm, einer Breite von 1 cm, und einer Höhe von 2.1 mm derart ausgelegt, dass eine große Diffusionsfläche zwischen dem Eluat und dem auf der Bodenfläche der Kammer in getrockneter Form vorliegenden analytspezifischen Bindungspartner (eingetrocknete Lösung des Δ⁹-THC spezifischen Antikörpers in 50 mM Carbonatpuffer, pH 6) zustande kam. Mikroskopische Untersuchungen zeigten, dass nach Inkubation der analythaltigen Probe mit dem analytspezifischen Bindungspartner keine Rückstände des getrockneten analytspezifischen Bindungspartners in der zweiten Kammer vorlagen.

Nach Beendigung der stationären Phase wurde das Reaktionsgemisch über ein mikrofluidisches Kapillarsystem auf einen Teststreifen überführt und der Chromatographieprozess hierdurch gestartet. Die vollständige Testentwicklung betrug 5 bis 10 Minuten. Die Ergebnisse der vier Bestimmungen, welche visuell oder unter Verwendung eines entsprechenden Auslesegerätes erfolgen kann, sind in Fig. 3 dargestellt.

Wie aus Fig. 3 hervorgeht, führen unterschiedliche Inkubationszeiten in der zweiten Kammer der mikrofluidischen Analysevorrichtung zu einer unterschiedlich starken Signalentwicklung an der Testlinie des chromatographischen Teststreifens. Die Skalierung der Signalintensität (y-Achse) umfasst 9 Einheiten, wobei Signale im Bereich von 0-3 als negativ, Signale im Bereich von 4-5 als schwach positiv, und Signale im Bereich von > 6 als positiv anzusehen sind. Die Zeit der Inkubation (x-Achse) ist in Minuten angegeben. Es zeigt sich, dass durch Einstellen einer Inkubationszeit von 2 Minuten die Sensitivität deutlich erhöht werden kann und bei ca. 3 Minuten ein Optimum erreicht.

### Beispiel 7: Bereitstellung definierter und konstanter Timerfunktionen

Zur Bereitstellung einer mikrofluidischen Analysevorrichtung mit definierten und konstanten Timerfunktionen wurde der pH-Indikator Bromthymolblau (Firma Sigma) in 1 mM Citratpuffer, pH 5.0 (Firma Sigma) gelöst und auf eine Konzentration von 1 mg/ml eingestellt. Diese Lösung wurde anschließend an einer vorbestimmten Position auf einen chromatographischen Teststreifen aufgebracht. Durch Aufbringen von 20 mM Tris-Puffer, pH 8.5 (Firma Sigma) auf ein Ende des Teststreifens wurde die Chromatographie gestartet.

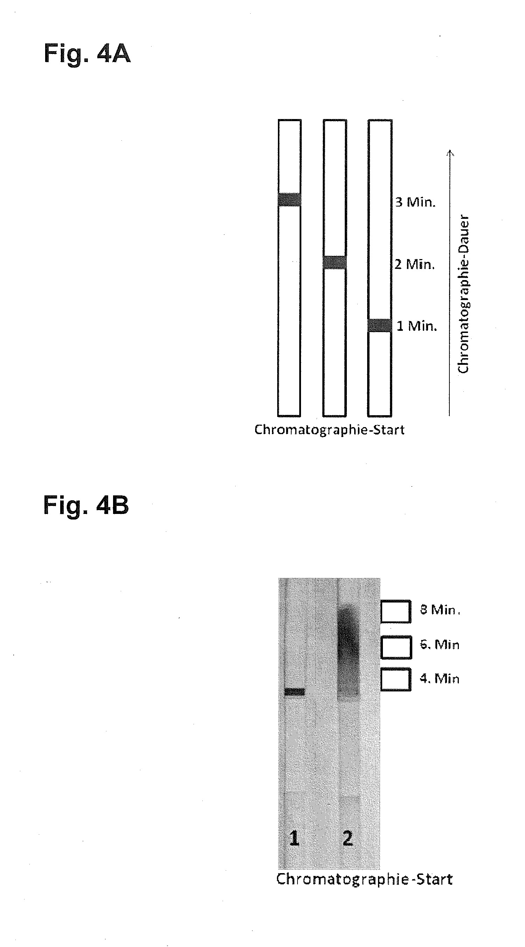

Nach Ablauf eines definierten Zeitraums erreichte das Laufmittel die mit Bromthymolblau markierte Zone des Teststreifens, wobei sich die Farbe des pH-Indikators von gelb nach blau änderte. Der Farbumschlag zeigt dem Anwender das Ende eines definierten Zeitraums an, welcher von der vorherigen Positionierung der Farbstofflösung auf dem Teststreifen abhängig ist. Fig. 4A zeigt drei Teststreifen, welche infolge unterschiedlicher Positionierung der Farbstofflösung einen Zeitraum von 1 Minute, 2 Minuten bzw. 3 Minuten definieren.

In einer Variante wurde als Farbstoff Methylblau (Firma Sigma) verwendet, welcher 1:10 in destilliertem Wasser angesetzt wurde. Diese Lösung wurde anschließend an einer vorbestimmten Position auf einen chromatographischen Teststreifen aufgebracht. Durch Aufbringen von destilliertem Wasser auf ein Ende des Teststreifens wurde die Chromatographie gestartet.

Nach einer gewissen Zeit erreichte das Laufmittel die mit Methylblau markierte Zone des Teststreifens, wobei der Farbstoff vom Laufmittel mittransportiert und bis zu einem Auslesefenster bewegt wird. Das Auslesefenster zeigt dem Anwender das Ende eines definierten Zeitraums an, welcher von der Positionierung des Auslesefensters auf dem Teststreifen abhängig ist. Fig. 4B zeigt einen derartigen Teststreifen vor Start der Chromatographie (1) bzw. nach einer Chromatographiedauer von etwa 8 Minuten (2).

## Patentansprüche

1. Verfahren zur Bestimmung eines Analyten, umfassend die Schritte:
(a) Bereitstellen einer mikrofluidischen Analysevorrichtung, umfassend zumindest einen ersten, einen zweiten und einen dritten Bereich, wobei der erste Bereich mit dem zweiten Bereich und der zweite Bereich mit dem dritten Bereich durch Mikrokanäle, Stufen, Verzweigungen oder/und Kammern verbunden ist,
(b) Aufnehmen einer den Analyten enthaltenden Probe von einer Probenoberfläche mit einem Probennahmeelement
(c) Einbringen des Probennahmeelements in den ersten Bereich der mikrofluidischen Analysevorrichtung und Eluieren des Analyten in dem ersten Bereich mit einem Elutionsmittel,
(d) Überführen des in Schritt (c) erhaltenen Eluats in den zweiten Bereich der mikrofluidischen Analysevorrichtung und Inkontaktbringen des Eluats mit einem in dem zweiten Bereich gelagerten analytspezifischen Bindungspartner für einen Zeitraum von mindestens 3 Sekunden in Abwesenheit eines zum Nachweis des Analyten dienenden Testelements, wobei eine Bindung des Analyten an den analytspezifischen Bindungspartner erfolgt,
(e) Überführen des in Schritt (d) erhaltenen Gemisches in den dritten Bereich der mikrofluidischen Analysevorrichtung und Inkontaktbringen des Gemisches mit mindestens einem in dem dritten Bereich gelagerten, zur Bestimmung des Analyten dienenden Testelement, und
(f) Bestimmen des Vorhandenseins oder/und der Menge des Analyten auf dem Testelement.

2. Verfahren nach Anspruch 1,
**dadurch gekennzeichnet,**
**dass** ein Probennahmeelement verwendet wird, welches einen Volumenindikator umfasst, wobei der Volumenindikator dem Anwender anzeigt, ob ein für die Bestimmung des Analyten ausreichendes Probenvolumen genommen wurde.

3. Verfahren nach Anspruch 1 oder 2,
**dadurch gekennzeichnet,**
**dass** das Elutionsmittel in dem Probennahmeelement oder/und in einem vierten Bereich der mikrofluidischen Analysevorrichtung gelagert wird.

4. Verfahren nach einem der Ansprüche 1 bis 3,
**dadurch gekennzeichnet,**
**dass** ein Elutionsmittel verwendet wird, welches
(a) eine wässrige Pufferlösung, und
(b) gegebenenfalls ein Protein, ein Proteingemisch, ein Kohlenhydrat, einen Zuckeralkohol, ein Detergenz oder/und ein organisches Lösungsmittel umfasst.

5. Verfahren nach einem der Ansprüche 1 bis 4,
**dadurch gekennzeichnet,**
**dass** als analytspezifischer Bindungspartner ein Antikörper oder ein funktionelles Antikörperfragment verwendet wird und gegebenenfalls eine optisch detektierbare Markierung, insbesondere eine Metallmarkierung, umfasst.

6. Verfahren nach einem der Ansprüche 1 bis 5,
**dadurch gekennzeichnet,**
**dass** der analytspezifische Bindungspartner bei Einbringen des in Schritt (c) erhaltenen Eluats in den zweiten Bereich der mikrofluidischen Analysevorrichtung gelöst und homogen in der Lösung verteilt wird.

7. Verfahren nach einem der Ansprüche 1 bis 6,
**dadurch gekennzeichnet,**
**dass** die Reaktion zwischen dem Analyten und dem analytspezifischen Bindungspartner in Schritt (d) für einen Zeitraum von etwa 5 Sekunden bis etwa 600 Sekunden, insbesondere von etwa 90 Sekunden bis etwa 240 Sekunden, durchgeführt wird

8. Verfahren nach einem der Ansprüche 1 bis 7,
**dadurch gekennzeichnet,**
**dass** die Reaktion zwischen dem Analyten und dem analytspezifischen Bindungspartner in Schritt (d) in stationärer Phase durchgeführt wird.

9. Verfahren nach einem der Ansprüche 1 bis 8,
**dadurch gekennzeichnet,**
**dass** als Testelement ein chromatographischer Teststreifen verwendet wird.

10. Verfahren nach einem der Ansprüche 1 bis 9,
**dadurch gekennzeichnet,**
**dass** die Bestimmung des Analyten in Schritt (f) optisch, insbesondere optisch-visuell, erfolgt.

11. Verfahren nach einem der Ansprüche 1 bis 10,
**dadurch gekennzeichnet,**
**dass** die Dauer des Schritts (d) oder/und des Schritts (f) über mindestens einen in die mikrofluidische Analysevorrichtung integrierten Zeitmesser, insbesondere zwei in die mikrofluidische Analysevorrichtung integrierte Zeitmesser, überwacht wird.

12. Verfahren nach einem der Ansprüche 1 bis 11,
**dadurch gekennzeichnet,**
**dass** als Analyt ein Amphetamin, ein Methamphetamin, ein Cannabinoid, insbesondere Δ⁹-Tetrahydrocannabinol, ein Opiat, insbesondere Morphin, Codein oder Dihydrocodein, ein Opioid, insbesondere Heroin, ein Tropan-Alkaloid, insbesondere Kokain, oder ein Benzodiazepin verwendet wird.

13. Verfahren nach einem der Ansprüche 1 bis 12,
**dadurch gekennzeichnet,**
**dass** als Probe ein Körperfluid, insbesondere Blut, Urin oder Speichel, verwendet wird.

14. Verfahren nach einem der Ansprüche 1 bis 13,
**dadurch gekennzeichnet,**
**dass** eine Probe mit einem Volumen von etwa 5 µl bis etwa 500 µl, insbesondere von etwa 30 µl bis etwa 150 µl, verwendet wird.

15. Kit, insbesondere zur Durchführung des Verfahrens nach einem der Ansprüche 1 bis 14, umfassend
(a) eine mikrofluidische Analysevorrichtung zur Bestimmung eines Analyten, umfassend:
(i) einen ersten Bereich, welcher zur Aufnahme eines Probennahmeelements mit hierauf aufgenommenem Analyten und zum Eluieren des Analyten von dem Probennahmeelement ausgebildet ist,
(ii) einen zweiten Bereich, welcher einen für den Analyten spezifischen Bindungspartner enthält und mit dem ersten Bereich durch Mikrokanäle, Stufen, Verzweigungen oder/und Kammern verbunden ist, wobei der zweite Bereich kein zum Nachweis des Analyten dienendes Testelement umfasst,
(iii) einen dritten Bereich, welcher mindestens ein Testelement zur Bestimmung des Vorhandenseins oder/und der Menge des Analyten umfasst und mit dem zweiten Bereich durch Mikrokanäle, Stufen, Verzweigungen oder/und Kammern verbunden ist,
(iv) gegebenenfalls einen vierten Bereich, welcher ein Elutionsmittel zum Eluieren des Analyten von dem Probennahmeelement umfasst und mit dem ersten Bereich in fluider Kommunikation steht,
(v) gegebenenfalls einen fünften Bereich, welcher mindestens einen Zeitmesser zur zeitlichen Überwachung der in der mikrofluidischen Analysevorrichtung ablaufenden Reaktionen umfasst, und
(vi) gegebenenfalls ein Gehäuse, und
(b) ein Probennahmeelement, welches in die mikrofluidische Analysevorrichtung aufgenommen werden kann und gegebenenfalls ein Elutionsmittel zum Eluieren des Analyten von dem Probennahmeelement umfasst.

## Claims

1. Method for determining an analyte, comprising the steps:
(a) providing a microfluidic analysis device, comprising at least a first, a second and a third region, the first region being connected to the second region via microchannels, stages, branches or/and chambers and the second region being connected to the third region via microchannels, stages, branches or/and chambers,
(b) receiving a sample containing the analyte from a sample surface by means of a sampling element,
(c) introducing the sampling element into the first region of the microfluidic analysis device and eluting the analyte in the first region by means of an eluent,
(d) transferring the eluate which was obtained in step (c) into the second region of the microfluidic analysis device, and bringing the eluate into contact with an analyte-specific binding partner, which is stored in the second region, for a period of at least 3 seconds, in the absence of a test element for the detection of the analyte, wherein the analyte binds to the analyte-specific binding partner,
(e) transferring the mixture which was obtained in step (d) into the third region of the microfluidic analysis device, and bringing the mixture into contact with at least one test element which is stored in the third region and serves for determining the analyte, and
(f) determining the presence or/and the amount of the analyte on the test element.

2. Method according to claim 1, **characterised in that** a sampling element which comprises a volume indicator is used, wherein the volume indicator shows the user whether sufficient sample volume has been taken for determining the analyte.

3. Method according to either claim 1 or claim 2, **characterised in that** the eluent is stored in the sampling element or/and in a fourth region of the microfluidic analysis device.

4. Method according to any one of claims 1 to 3, **characterised in that** an eluent is used which comprises
(a) an aqueous buffer solution and
(b) optionally a protein, a protein mixture, a carbohydrate, a sugar alcohol, a detergent or/and an organic solvent.

5. Method according to any one of claims 1 to 4, **characterised in that** an antibody or a functional antibody fragment, which optionally comprises an optically detectable label, in particular a metal label, is used as the analyte-specific binding partner.

6. Method according to any one of claims 1 to 5, **characterised in that** the analyte-specific binding partner is dissolved and homogeneously distributed in the solution when the eluate which was obtained in step (c) is introduced into the second region of the microfluidic analysis device.

7. Method according to any one of claims 1 to 6, **characterised in that** the reaction between the analyte and the analyte-specific binding partner in step (d) is carried out for a period of approximately 5 seconds to approximately 600 seconds, in particular for approximately 90 seconds to approximately 240 seconds.

8. Method according to any one of claims 1 to 7, **characterised in that** the reaction between the analyte and the analyte-specific binding partner in step (d) is carried out in a stationary phase.

9. Method according to any one of claims 1 to 8, **characterised in that** a chromatographic test strip is used as the test element.

10. Method according to any one of claims 1 to 9, **characterised in that** the determination of the analyte in step (f) occurs optically, in particularly optically by eye.

11. Method according to any one of claims 1 to 10, **characterised in that** the duration of step (d) or/and of step (f) is monitored by means of at least one timing device which is integrated into the microfluidic analysis device, in particular two timing devices which are integrated into the microfluidic analysis device.

12. Method according to any one of claims 1 to 11, **characterised in that** an amphetamine, a methamphetamine, a cannabinoid, in particular Δ⁹-tetrahydrocannabinol, an opiate, in particular morphine, codeine or dihydrocodeine, an opioid, in particular heroin, a tropane alkaloid, in particular cocaine, or a benzodiazepine is used as analyte.

13. Method according to any one of claims 1 to 12, **characterised in that** a bodily fluid, in particular blood, urine or saliva, is used as a sample.

14. Method according to any one of claims 1 to 13, **characterised in that** a sample having a volume of approximately 5 µl to approximately 500 µl, in particular of approximately 30 µl to approximately 150 µl is used.

15. Kit, in particular for carrying out the method according to any one of claims 1 to 14, comprising:
(a) a microfluidic analysis device for determining an analyte, comprising:
(i) a first region which is configured for introducing a sampling element with an analyte received thereon and for eluting the analyte from the sampling element,
(ii) a second region which comprises a binding partner which is specific to the analyte and is connected to the first region via microchannels, stages, branches or/and chambers, the second region not comprising a test element which is used for detecting the analyte,
(iii) a third region which comprises at least one test element for determining the presence or/and the amount of the analyte and is connected to the second region via microchannels, stages, branches or/and chambers,
(iv) optionally a fourth region which comprises an eluent for eluting the analyte from the sampling element and which is in fluid communication with the first region,
(v) optionally a fifth region which comprises at least one timing device for temporally monitoring the reactions taking place in the analysis device, and
(vi) optionally a housing, and
(b) a sampling element which can be received in the microfluidic analysis device and which optionally comprises an eluent for eluting the analyte from the sampling element.

## Revendications

1. Procédé servant à déterminer un analyte, comprenant les étapes suivantes :
(a) mise à disposition d'un dispositif d'analyse microfluidique, comprenant au moins une première zone, une deuxième zone et une troisième zone, sachant que la première zone est reliée à la deuxième zone par des micro-canaux, des passages, des branchements et/ou des chambres et que la deuxième zone est reliée à la troisième zone par des micro-canaux, des passages, des branchements et/ou des chambres ;
(b) prélèvement d'un échantillon contenant l'analyte d'une surface d'échantillon à l'aide d'un élément de prélèvement d'échantillon
(c) introduction de l'élément de prélèvement d'échantillon dans la première zone du dispositif d'analyse microfluidique et élution de l'analyte dans la première zone à l'aide d'un éluant ;
(d) transfert de l'éluat obtenu à l'étape (c) dans la deuxième zone du dispositif d'analyse microfluidique et mise en contact de l'éluat avec un partenaire de liaison spécifique à l'analyte stocké dans la deuxième zone pour une durée d'au moins 3 secondes, en l'absence d'un élément de test servant pour la détection d'analyte, dans lequel une liaison de l'analyte se produit avec le partenaire de liaison spécifique à l'analyte ;
(e) transfert du mélange obtenu à l'étape (d) dans la troisième zone du dispositif d'analyse microfluidique et mise en contact du mélange avec au moins un élément de test stocké dans la troisième zone, servant pour la détermination de l'analyte; et
(f) détermination de la présence et/ou de la quantité de l'analyte sur l'élément de test.

2. Procédé selon la revendication 1,
**caractérisé en ce**
**qu'**est utilisé un élément de prélèvement d'échantillon, qui comprend un indicateur de volume, dans lequel l'indicateur de volume indique à l'utilisateur si un volume d'échantillon suffisant a été prélevé pour la détermination de l'analyte .

3. Procédé selon la revendication 1 ou 2,
**caractérisé en ce**
**que** l'éluant est stocké dans l'élément de prélèvement d'échantillon et/ou dans une quatrième zone du dispositif d'analyse microfluidique.

4. Procédé selon l'une quelconque des revendications 1 à 3,
**caractérisé en ce**
**qu'**est utilisé un éluant, qui comprend
(a) une solution tampon aqueuse, et
(b) optionellement, une protéine, un mélange de protéines, un glucide, un alcool de sucre, un détergent et/ou un solvant organique.

5. Procédé selon l'une quelconque des revendications 1 à 4,
**caractérisé en ce**
**qu'**est utilisé en tant que partenaire de liaison spécifique à l'analyte un anticorps ou un fragment d'anticorps fonctionnel, et en ce qu'il comprend optionellement un marquage détectable optiquement, en particulier un marquage métallique.

6. Procédé selon l'une quelconque des revendications 1 à 5,
**caractérisé en ce**
**que** le partenaire de liaison spécifique à l'analyte est dissous lors de l'introduction de l'éluat obtenu à l'étape (c) dans la deuxième zone du dispositif d'analyse microfluidique et est réparti de manière homogène dans la solution.

7. Procédé selon l'une quelconque des revendications 1 à 6,
**caractérisé en ce**
**que** la réaction entre l'analyte et le partenaire de liaison spécifique à l'analyte est réalisée à l'étape (d), pour une durée allant d'environ 5 à environ 600 secondes, en particulier allant d'environ 90 secondes à environ 240 secondes.

8. Procédé selon l'une quelconque des revendications 1 à 7,
**caractérisé en ce**
**que** la réaction entre l'analyte et le partenaire de liaison spécifique à l'analyte est réalisée à l'étape (d) en phase stationnaire.

9. Procédé selon l'une quelconque des revendications 1 à 8,
**caractérisé en ce**
**qu'**est utilisé en tant qu'élément de test une bandelette de test chromatographique.

10. Procédé selon l'une quelconque des revendications 1 à 9,
**caractérisé en ce**
**que** la détermination de l'analyte est effectuée à l'étape (f) de manière optique, en particulier de manière optique et visuelle.

11. Procédé selon l'une quelconque des revendications 1 à 10,
**caractérisé en ce**
**que** la durée de l'étape (d) et/ou de l'étape (f) est contrôlée par l'intermédiaire d'au moins un chronomètre intégré dans le dispositif d'analyse microfluidique, en particulier deux chronomètres intégrés dans le dispositif d'analyse microfluidique.

12. Procédé selon l'une quelconque des revendications 1 à 11,
**caractérisé en ce**
**qu'**est utilisé en tant qu'analyte une amphétamine, une méthamphétamine, un cannabinoïde, en particulier un Δ⁹-tétrahydrocannabinol, un opiacé, en particulier de la morphine, de la codéine ou de la dihydrocodéine, un opioïde, en particulier de l'héroïne, un alcaloïde tropanique, en particulier de la cocaïne, ou une benzodiazépine.

13. Procédé selon l'une quelconque des revendications 1 à 12,
**caractérisé en ce**
**qu'**est utilisé en tant qu'échantillon, un fluide corporel, en particulier du sang, de l'urine ou de la salive.

14. Procédé selon l'une quelconque des revendications 1 à 13,
**caractérisé en ce**
**qu'**est utilisé un échantillon présentant un volume allant d'environ 5 µl à environ 500 µl, en particulier allant d'environ 30 µl à environ 150 µl.

15. Kit, en particulier pour l'exécution du procédé selon l'une quelconque des revendications 1 à 14, comprenant
(a) un dispositif d'analyse microfluidique pour la détermination d'un analyte, comprenant :
(i) une première zone, qui est adaptée pour recevoir un élément de prélèvement d'échantillon avec dessus l'analyte qui a été prélevé et pour l'élution de l'analyte de l'élément de prélèvement d'échantillon ;
(ii) une deuxième zone, qui contient un partenaire de liaison spécifique à l'analyte et qui est reliée à la première zone par des micro-canaux, des passages, des branchements et/ou des chambres, la deuxième zone ne contenant aucun élément de test pour la détection de l'analyte;
(iii) une troisième zone, qui comprend au moins un élément de test pour la détermination de la présence et/ou de la quantité de l'analyte et est reliée à la deuxième zone par des micro-canaux, des passages, des branchements et/ou des chambres,
(iv) optionnellement une quatrième zone, qui comprend un éluant pour l'élution de l'analyte de l'élément de prélèvement d'échantillon et qui se trouve en communication fluidique avec la première zone,
(v) optionnellement une cinquième zone, qui comprend au moins un chronomètre pour le contrôle de la durée des réactions se déroulant dans le dispositif d'analyse microfluidique, et
(vi) optionnellement un boîtier, et
(b) un élément de prélèvement d'échantillon, qui peut être introduit dans le dispositif d'analyse microfluidique et qui comprend optionnellement un éluant pour l'élution de l'analyte de l'élément de prélèvement d'échantillon.
